# EUROPEAN PATENT APPLICATION

(11) **EP 4 349 811 A1**
(43) Date of publication of application: **10.04.2024**
(21) Application number: 22815991.9
(22) Date of filing: 27.05.2022
(51) Int. Cl.: C07D 209/86, C09K 11/06, H01L 51/50, H05B 33/10

(54) **DEUTERIDE AND ORGANIC ELECTROLUMINESCENT ELEMENT**

(30) Priority: 31.05.2021 JP 2021091968
(71) Applicant: NIPPON STEEL Chemical & Material Co., Ltd., Tokyo 103-0027 (JP)
(72) Inventor: IKENAGA Yuji, Tokyo 103-0027 (JP); KAI Takahiro, Tokyo 103-0027 (JP); HAYASHI Kentaro, Tokyo 103-0027 (JP); SAKAI Mitsuru, Tokyo 103-0027 (JP); SHIMAMOTO Yuya, Tokyo 103-0027 (JP)
(74) Representative: Kraus & Lederer PartGmbB
(86) International application number: PCT/JP2022/021713
(87) International publication number: WO 2022/255241

(57) **Abstract**

To provide a practically useful organic EL device having a low driving voltage and also having a high efficiency and a long lifetime, and a deuteride suitable therefor. A deuteride of a compound represented by the following general formula (1), in which a rate of deuteration of hydrogen atoms on two carbazole rings in the compound is 30% or more, wherein Ar¹ and Ar² each independently represent a substituted or unsubstituted aromatic hydrocarbon group having 6 to 30 carbon atoms, or a substituted or unsubstituted linked aromatic group in which two to five of these aromatic rings are linked to each other, and aromatic hydrocarbon groups in the case of these aromatic rings linked are the same as or different from each other.

## Description

### Technical Field

The present invention relates to a deuteride and an organic electroluminescent device in which the deuteride is used.

### Background Art

Application of a voltage to an organic electroluminescent element or device (referred to as an organic EL device.) allows injection of holes and electrons from an anode and a cathode, respectively, into a light-emitting layer. Then, in the light-emitting layer, injected holes and electrons recombine to generate excitons. At this time, according to statistical rules of electron spins, singlet excitons and triplet excitons are generated at a ratio of 1:3. Regarding a fluorescence-emitting organic EL device using light emission from singlet excitons, it is said that the internal quantum efficiency thereof has a limit of 25%. Meanwhile, regarding a phosphorescent organic EL device using light emission from triplet excitons, it is known that intersystem crossing is efficiently performed from singlet excitons, the internal quantum efficiency is enhanced to 100%.

However, a technical object of a phosphorescent organic EL device is to increase the lifetime.

Moreover, highly efficient organic EL devices utilizing delayed fluorescence have been developed recently. For example, Patent Literature 1 discloses an organic EL device utilizing a TTF (Triplet-Triplet Fusion) mechanism, which is one of delayed fluorescence mechanisms. The TTF mechanism utilizes a phenomenon in which singlet excitons are generated due to collision of two triplet excitons, and it is thought that the internal quantum efficiency can be theoretically raised to 40%. However, since the efficiency is lower compared to phosphorescent organic EL devices, further improvement in efficiency is required.

Meanwhile, patent Literature 2 discloses an organic EL device utilizing a TADF (Thermally Activated Delayed Fluorescence) mechanism. The TADF mechanism utilizes a phenomenon in which reverse intersystem crossing from triplet excitons to singlet excitons is generated in a material having a small energy difference between a singlet level and a triplet level, and it is thought that the internal quantum efficiency can be theoretically raised to 100%. However, there is a demand for further improvement in lifetime characteristics, as in a phosphorescent device.

### Citation List

### Patent Literature

Patent Literature 1: WO2010/134350 A
Patent Literature 2: WO2011/070963 A
Patent Literature 3: WO2008/056746 A
Patent Literature 4: KR2013/132226 A
Patent Literature 5: JP2003-133075 A
Patent Literature 6: US2014/0374728 A
Patent Literature 7: US2014/0197386 A
Patent Literature 8: US2015/0001488 A
Patent Literature 9: US2008/0286605 A
Patent Literature 10: WO2016/194604 A
Patent Literature 11: WO2018/198844 A
Patent Literature 12: WO2012/153725 A

Patent Literatures 3 and 4 disclose use of an indolocarbazole compound as a host material. Patent Literature 5 discloses use of a biscarbazole compound as a host material.

Patent Literature 6 discloses use of a biscarbazole compound as a mixed host. Patent Literatures 7 and 8 disclose use of an indolocarbazole compound and a biscarbazole compound, as a mixed host.

Patent Literature 9 discloses use of a deuterated carbazole compound as a host material.

Patent Literatures 10 and 11 disclose use of a host material in which a plurality of indolocarbazole compounds is premixed.

Patent Literature 12 discloses use of a deuterated carbazole compound as a hole transport material.

However, none of these can be said to be sufficient in that a device achieves a reduction in driving voltage, an enhancement in luminous efficiency, and an increase in lifetime, and further improvement is desired.

### Summary of Invention

### Technical Problem

In order to apply organic EL devices to display devices such as flat panel displays, it is necessary to improve the luminous efficiency of devices and at the same time, to sufficiently secure the lifetime characteristics of the devices. In view of the above circumstances, an object of the present invention is to provide a practically useful organic EL device having a low driving voltage and also having a high efficiency and a long lifetime, and a compound suitable therefor.

As a result of intensive studies, the present inventors have found that excellent characteristics are exhibited by use of a deuteride of a compound represented by the following general formula (1) in which the rate of deuteration of hydrogen atoms on two carbazole rings in the compound is 30% or more, for an organic EL device, and have completed the present invention.

The present invention relates to a deuteride of a compound represented by general formula (1), wherein a rate of deuteration of hydrogen atoms on two carbazole rings in the compound is 30% or more:

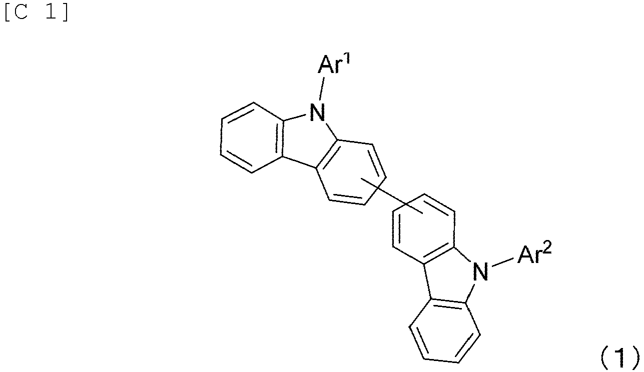

wherein Ar¹ and Ar² each independently represent a substituted or unsubstituted aromatic hydrocarbon group having 6 to 30 carbon atoms, or a substituted or unsubstituted linked aromatic group in which two to five of these aromatic rings are linked to each other, and aromatic hydrocarbon groups linked to each other are the same as or different from each other.

In the deuteride, the rate of deuteration of hydrogen atoms on two carbazole rings is preferably 40% or more, the rate of deuteration of hydrogen atoms on two carbazole rings is more preferably 50% or more, and the rate of deuteration of hydrogen atoms on two carbazole rings is further preferably 70% or more.

The compound represented by the general formula (1) is preferably a compound represented by the following formula (2): 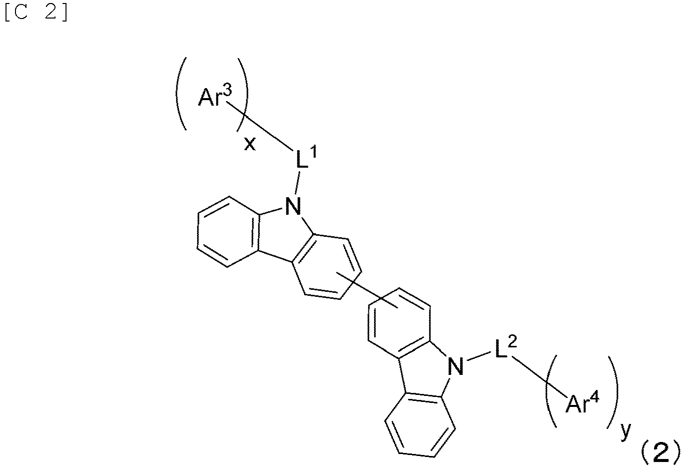 wherein Ar³ and Ar⁴ each independently represent a substituted or unsubstituted aromatic hydrocarbon group having 6 to 14 carbon atoms, or a substituted or unsubstituted linked aromatic group in which two of these aromatic rings are linked to each other, and aromatic hydrocarbon groups linked to each other are the same as or different from each other; L¹ and L² represent a substituted or unsubstituted phenylene group; and x and y each independently represent an integer of 0 to 2, preferably each independently represent 0 to 1.

The present invention relates to a mixture of the deuteride of a compound represented by the general formula (1) in which the rate of deuteration of hydrogen atoms on two carbazole rings in the compound is 30% or more, and a compound represented by the following general formula (3).

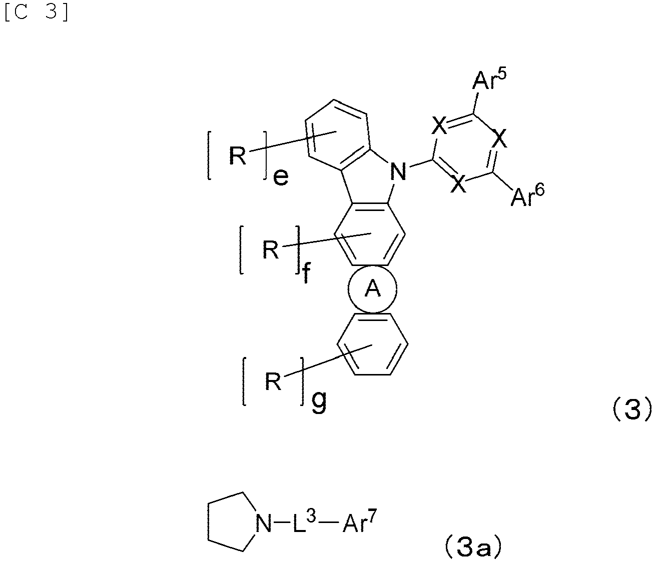

In the general formula (3), a ring A is a heterocycle fused to two adjacent rings at any positions and represented by formula (3a). Ar⁵ and Ar⁶ each independently represent hydrogen, a substituted or unsubstituted aromatic hydrocarbon group having 6 to 30 carbon atoms, a substituted or unsubstituted aromatic heterocyclic group having 3 to 17 carbon atoms, or a substituted or unsubstituted linked aromatic group in which two to five of these aromatic rings are linked to each other, and aromatic hydrocarbon groups or aromatic heterocyclic groups in the case of these aromatic rings linked are the same as or different from each other. Ar⁷ represents a substituted or unsubstituted aromatic hydrocarbon group having 6 to 30 carbon atoms, a substituted or unsubstituted aromatic heterocyclic group having 3 to 17 carbon atoms, or a substituted or unsubstituted linked aromatic group in which two to five of these aromatic rings are linked to each other, and aromatic hydrocarbon groups or aromatic heterocyclic groups in the case of these aromatic rings linked are the same as or different from each other. L³ represents a direct bond or a substituted or unsubstituted aromatic hydrocarbon group having 6 to 18 carbon atoms. Each R independently represents an aliphatic hydrocarbon group having 1 to 10 carbon atoms. e to g represent the number of substitutions, e and g represent an integer of 0 to 4, and f represents an integer of 0 to 2. X represents N, C-H or C-Ar⁸ and at least one thereof represents N. Each Ar⁸ independently represents a substituted or unsubstituted aromatic hydrocarbon group having 6 to 30 carbon atoms, a substituted or unsubstituted aromatic heterocyclic group having 3 to 17 carbon atoms, or a substituted or unsubstituted linked aromatic group in which two to five of these aromatic rings are linked to each other, and aromatic hydrocarbon groups or aromatic heterocyclic groups in the case of these aromatic rings linked are the same as or different from each other. In addition, e and g preferably represent an integer of 0 to 2, and all of e to g more preferably represent 0.

The difference in 50% weight reduction temperatures of the deuteride of a compound represented by the general formula (1) in which the rate of deuteration of hydrogen atoms on two carbazole rings in the compound is 30% or more, and the compound represented by the general formula (3), is preferably within 20°C, more preferably within 15°C.

The mixture is preferably a mixture in which the proportion of the compound represented by the general formula (3) is 20 wt% or more and 70 wt% or less.

The present invention relates to an organic EL device comprising a plurality of organic layers between an anode and a cathode, wherein at least one of the organic layers contains the deuteride of a compound represented by the general formula (1) in which the rate of deuteration of hydrogen atoms on two carbazole rings in the compound is 30% or more, or a mixture of the deuteride of a compound represented by the general formula (1), and the compound represented by the general formula (3).

The organic layer containing the deuteride of a compound represented by the general formula (1), or the mixture of the deuteride of the compound represented by the general formula (1) and the compound represented by the general formula (3) is at least one selected from the group consisting of a light-emitting layer, a hole injection layer, a hole transport layer, an electron transport layer, an electron injection layer, a hole blocking layer and an electron blocking layer, more preferably a light-emitting layer. When the organic layer containing the deuteride or the mixture is a light-emitting layer, the light-emitting layer more preferably contains at least one light-emitting dopant.

The present invention relates to a method for producing an organic EL device comprising a plurality of organic layers between an anode and a cathode, wherein one of the organic layers is a light-emitting layer, and the light-emitting layer is produced by providing a mixture of the deuteride of a compound represented by the general formula (1) in which the rate of deuteration of hydrogen atoms on two carbazole rings in the compound is 30% or more, and the compound represented by the general formula (3), and using the mixture for vapor-deposition from one vapor deposition source.

### Advantageous Effect of Invention

The deuteride of the present invention can be used as a material for an organic EL device, has a structure represented by the general formula (1), and has deuterium on a carbazole ring, and therefore it is supposed that the number of substitutions and the substitution position(s) can be appropriately designed to thereby allow for enhancements in stabilities of an excited state and an ionic state and control of charge injection/transport properties at a high level. An organic EL device in which the deuteride is used is thus considered to have a long lifetime and a low voltage, and exhibit characteristics at a practical use level.

### Brief Description of Drawing

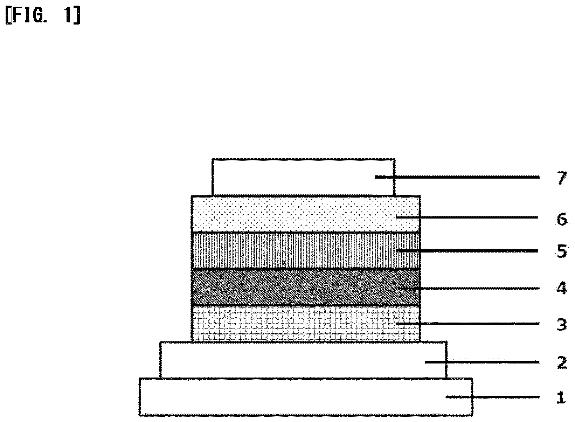

[Figure 1] Figure 1 is a cross-sectional view showing one structure example of an organic EL device.

### Description of Embodiments

The present invention relates to a deuteride of compound represented by the general formula (1) in which the rate of deuteration of hydrogen atoms on two carbazole rings in the compound is 30% or more. In the general formula (1), Ar¹ and Ar² each independently represent a substituted or unsubstituted aromatic hydrocarbon group having 6 to 30 carbon atoms, or a substituted or unsubstituted linked aromatic group in which two to five of these aromatic rings are linked to each other, preferably represent a substituted or unsubstituted aromatic hydrocarbon group having 6 to 24 carbon atoms, or a substituted or unsubstituted linked aromatic group in which two to five of these aromatic hydrocarbon groups are linked to each other, more preferably represent a substituted or unsubstituted aromatic hydrocarbon group having 6 to 18 carbon atoms, or a substituted or unsubstituted linked aromatic group in which two to five of these aromatic hydrocarbon groups are linked to each other. When the aromatic rings are linked, the aromatic hydrocarbon groups are the same as or different from each other.

Specific examples of the unsubstituted aromatic hydrocarbon group or linked aromatic group for Ar¹ or Ar² include a group generated by removing one hydrogen from benzene, naphthalene, acenaphthene, acenaphthylene, azulene, anthracene, chrysene, pyrene, phenanthrene, triphenylene, fluorene, benzo[a]anthracene, tetracene, pentacene, hexacene, coronene, heptacene, or compounds in which two to five of these are linked to each other. Preferred examples include a group generated from benzene, naphthalene, acenaphthene, acenaphthylene, azulene, anthracene, chrysene, pyrene, phenanthrene, triphenylene, fluorene, benzo[a]anthracene, tetracene, or compounds in which two to five of these are linked to each other. More preferred is a phenyl group, a biphenyl group, or a terphenyl group. The terphenyl group may be linked linearly or branched.

The unsubstituted aromatic hydrocarbon group or linked aromatic group may each have a substituent. In the case of having a substituent, the substituent is preferably halogen, a cyano group, a triarylsilyl group, an alkenyl group having 2 to 5 carbon atoms, an alkoxy group having 1 to 5 carbon atoms or a diarylamino group having 12 to 44 carbon atoms.

Note that the number of substituents is 0 to 5 and preferably 0 to 2. When an aromatic hydrocarbon group or a linked aromatic group has a substituent, the calculation of the number of carbon atoms does not include the number of carbon atoms of the substituent. However, it is preferred that the total number of carbon atoms including the number of carbon atoms of substituents satisfy the above range.

Specific examples of the substituent include bromo, fluorine, cyano, triphenylsilyl, vinyl, propenyl, butenyl, pentenyl, methoxy, ethoxy, propoxy, butoxy, pentoxy, diphenylamino, naphthylphenylamino, dinaphthylamino, dianthranylamino, diphenanthrenylamino, and dipyrenylamino. Preferred examples thereof include cyano, vinyl, propenyl, butenyl, pentenyl, methoxy, ethoxy, propoxy, butoxy, and pentoxy.

In the present specification, the linked aromatic group refers to an aromatic group in which the carbon atoms of the aromatic rings in two or more aromatic groups are linked to each other by a single bond. The linked aromatic group may be linear or branched. The linkage position in linking of benzene rings may be any of the ortho-, meta-, and para-positions, and is preferably the para-position or the meta-position. The aromatic group may be an aromatic hydrocarbon group or an aromatic heterocyclic group, and the plurality of aromatic groups may be the same or different.

The present invention relates to a deuteride of compound represented by the general formula (1) in which the rate of deuteration of hydrogen atoms on two carbazole rings in the compound is 30% or more. The rate of deuteration of hydrogen atoms on two carbazole rings is preferably 40% or more, the rate of deuteration of hydrogen atoms on two carbazole rings is more preferably 50% or more, and the rate of deuteration of hydrogen atoms on two carbazole rings is further preferably 70% or more.

A preferred aspect of the general formula (1) is general formula (2). Ar³ and Ar⁴ each independently represent a substituted or unsubstituted aromatic hydrocarbon group having 6 to 14 carbon atoms, or a substituted or unsubstituted linked aromatic group in which two of these aromatic rings are linked to each other, and aromatic hydrocarbon groups in the case of these aromatic rings linked are the same as or different from each other. L¹ and L² represent a substituted or unsubstituted phenylene group.

Specific examples of the unsubstituted aromatic hydrocarbon group having 6 to 14 carbon atoms or the linked aromatic group in which two of these aromatic rings are linked to each other include a group generated by removing one hydrogen from benzene, naphthalene, acenaphthene, acenaphthylene, azulene, anthracene, or compounds in which two of these are linked to each other. Preferred is a phenyl group or a biphenyl group.

L¹ and L² represent a substituted or unsubstituted phenylene group. The phenylene group may be bound at any of ortho-, meta-, and para-positions, and is preferably bound at the meta- or para-position.

When Ar³, Ar⁴, L¹, or L² is an aromatic hydrocarbon group, a linked aromatic group, or a phenylene group, the substituent is the same as in Ar¹ and Ar².

Any hydrogen in the compound used in the present invention may be deuterium. In other words, not only hydrogen on two carbazole rings in the compound represented by the general formula (1) or (2), but also hydrogen on aromatic rings in Ar¹, Ar², Ar³, Ar⁴, L¹ and L², hydrogen in other substituent, and furthermore hydrogen on an aromatic ring fused via a ring A and hydrogen in the substituent in the general formula (3) may be partially or fully deuterium. Herein, the rate of deuteration in the general formula (1) represents the rate of deuteration of hydrogen on two carbazole rings. The deuteride of the present invention encompasses both a single deuteride of a compound represented by the general formula (1) and a mixture of deuterides of two or more compounds represented by the general formula (1). In other words, in a case where the rate of deuteration is specifically described, a rate of deuteration of hydrogen on two carbazole rings, of 50%, means that seven hydrogen atoms on average, among fourteen hydrogen atoms, are substituted with deuterium, in which the deuteride may be a single compound or may be a mixture of compounds different in rate of deuteration.

The rate of deuteration can be determined by mass analysis or a proton nuclear magnetic resonance method. For example, when the rate is determined by a proton nuclear magnetic resonance method, a measurement sample is first prepared by adding and dissolving a compound and an internal standard material to and in a deuterated solvent, and the proton concentration [mol/g] in the compound included in the measurement sample is calculated from the ratio between integrated intensities derived from the internal standard material and the compound. Next, the ratio between the proton concentration in a deuterated compound and the proton concentration of the corresponding non-deuterated compound is calculated and subtracted from 1, and thus the rate of deuteration in the deuterated compound can be calculated. The rate of deuteration in a partial structure can be calculated by the same procedure, from the integrated intensity with respect to a chemical shift assigned to an objective partial structure.

A preferred aspect of the general formulas (1) and (2) is the following formula (2a): 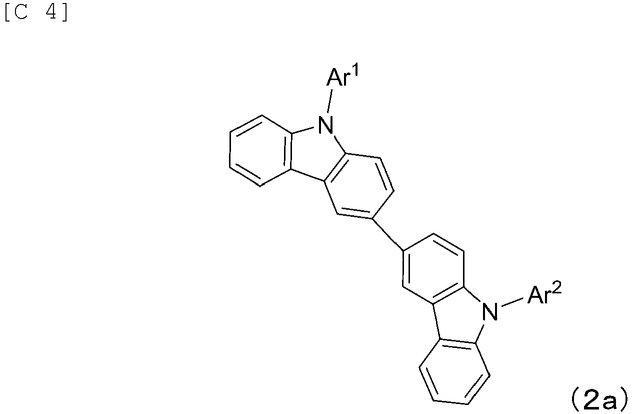 wherein Ar¹ and Ar² have the same meaning as in the general formula (1).

Specific examples of the deuteride of a compound represented by the general formula (1) in which the rate of deuteration of hydrogen atoms on two carbazole rings in the compound is 30% or more are shown below, but are not limited to these exemplified compounds. The numbers of substitutions with deuterium (D), for example, l, m, n, o, p, q, r, and s, in the following structural formula mean the average numbers, and are varied depending on the rate of deuteration (D-conversion rate).

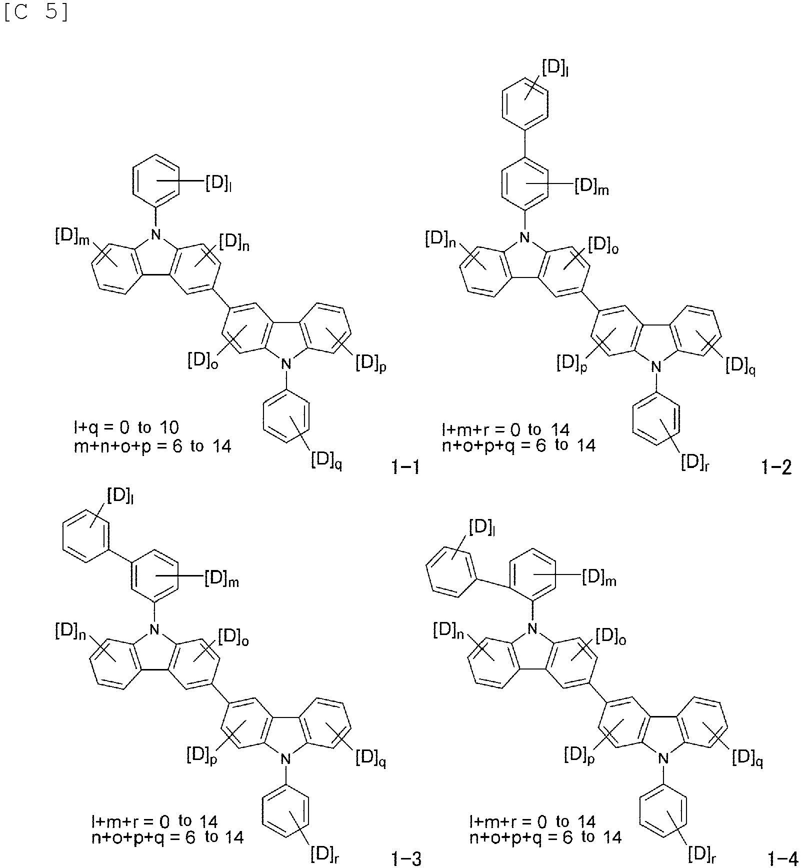

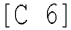

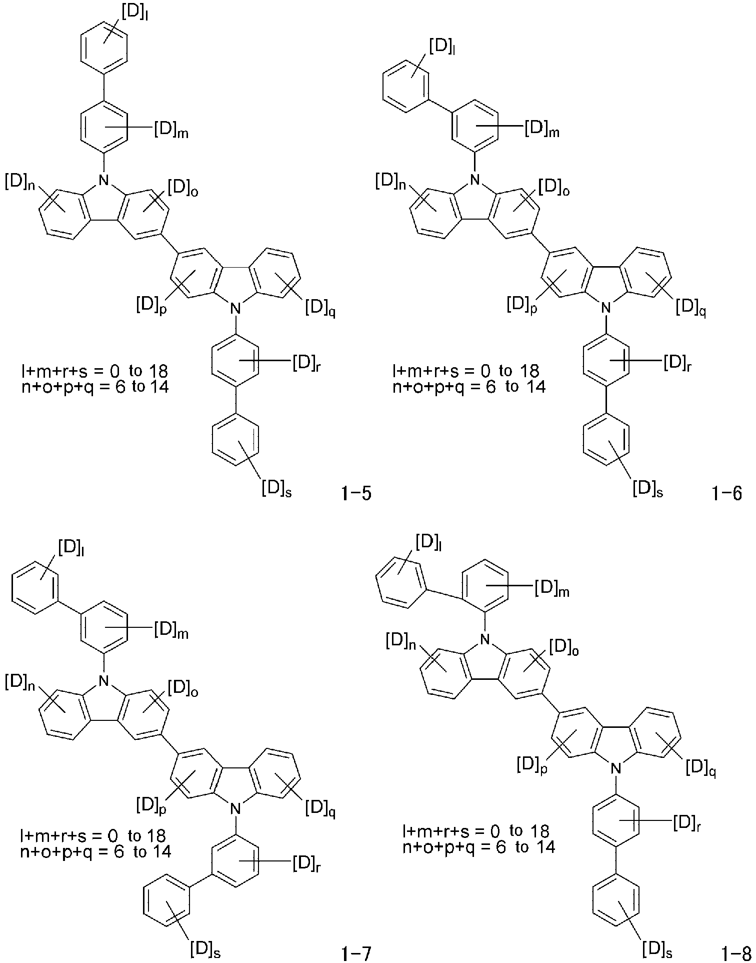

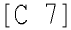

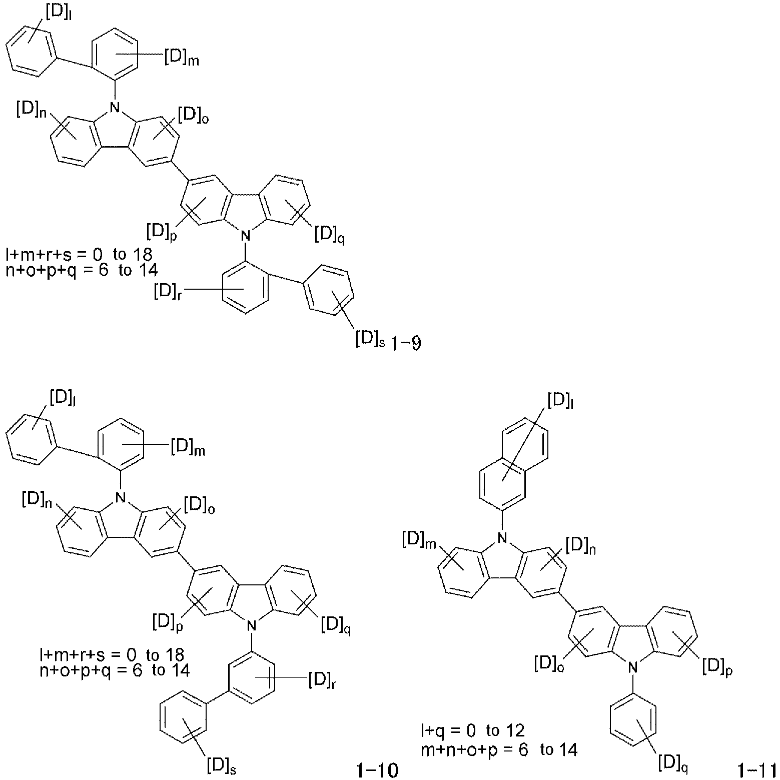

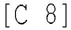

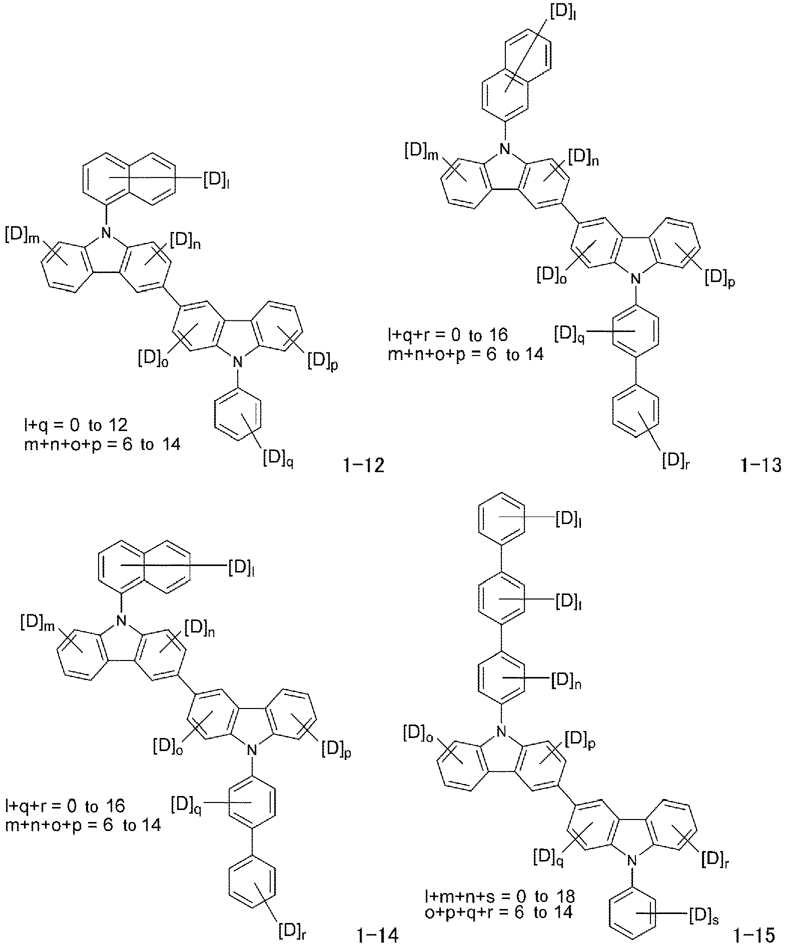

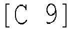

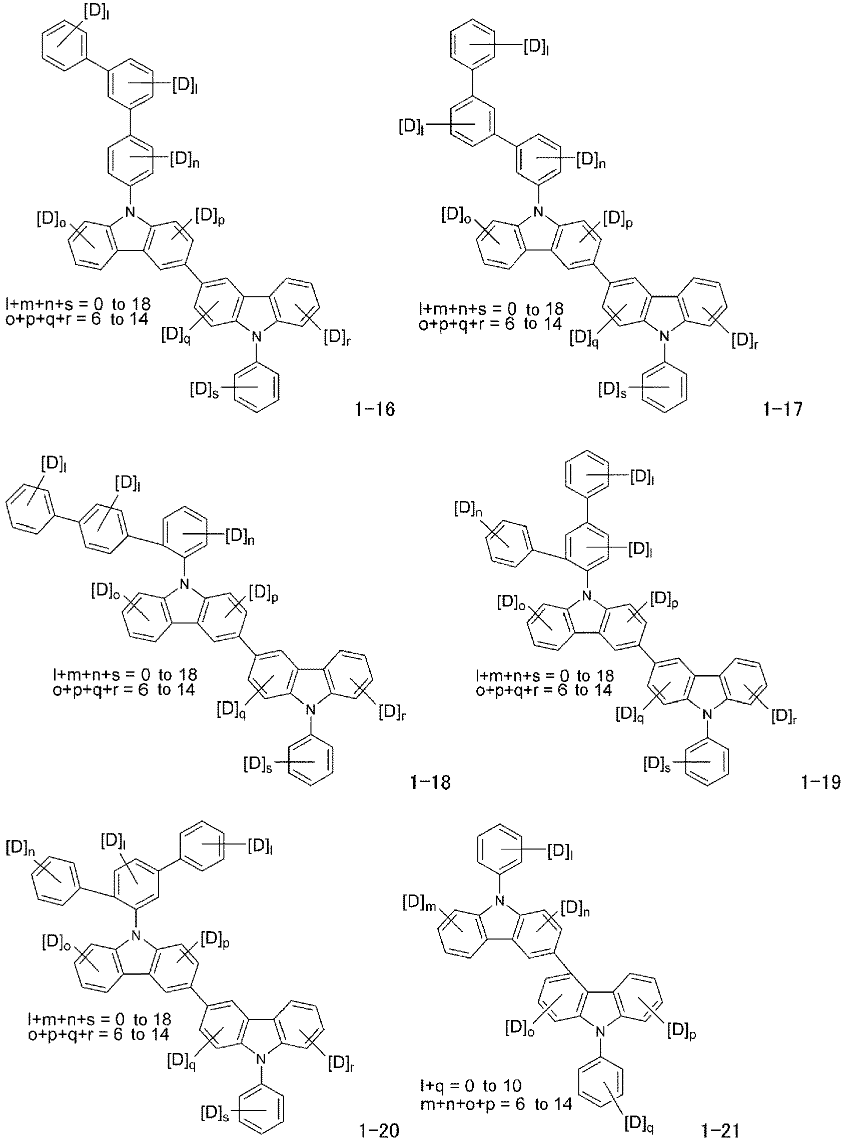

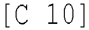

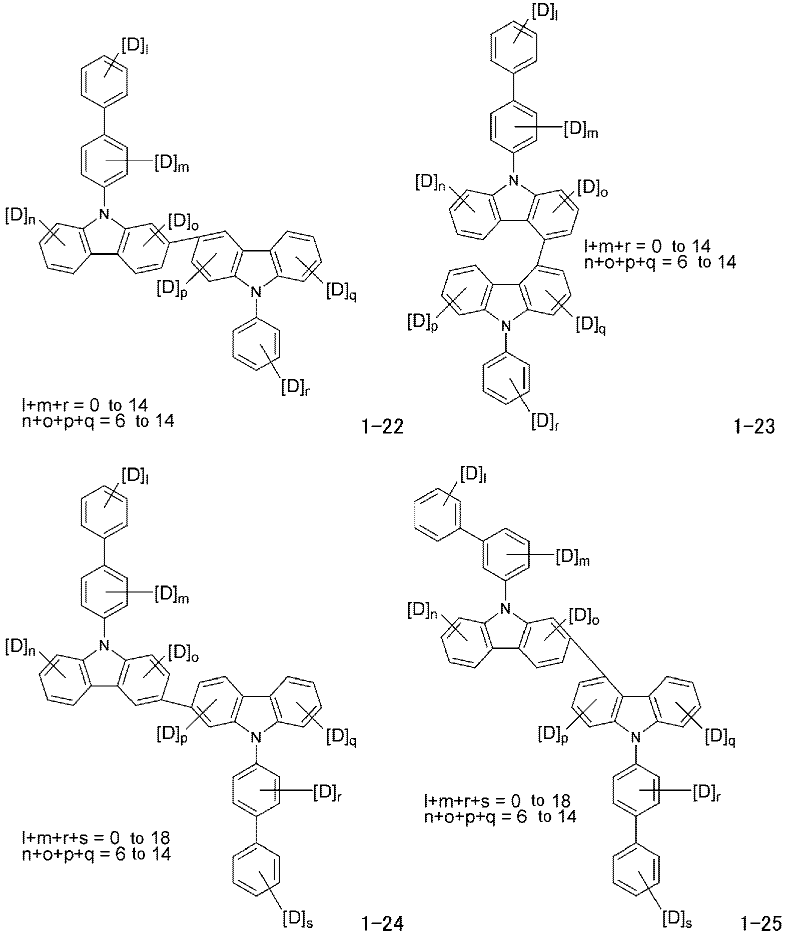

In the general formula (3), a ring A is a 5-membered heterocycle represented by formula (3a) and is fused to two adjacent rings at any positions, but is not fused at a side containing N. Hence, an indolocarbazole ring has some isomeric structures, but the number of the structures is restricted.

Each X independently represents N, C-H or C-Ar⁸ and at least one thereof represents N. Preferably, two or more X represent N. More preferably, all of X represent N.

Each R independently represents an aliphatic hydrocarbon group having 1 to 10 carbon atoms. In addition, e to g represent the number of substitutions, e and g represent an integer of 0 to 4, and f represents an integer of 0 to 2. e and g preferably represent an integer of 0 to 2, and all of e to g more preferably represent 0.

Each Ar⁵ and Ar⁶ independently represent hydrogen, a substituted or unsubstituted aromatic hydrocarbon group having 6 to 30 carbon atoms, a substituted or unsubstituted aromatic heterocyclic group having 3 to 17 carbon atoms, or a substituted or unsubstituted linked aromatic group in which two to five of these aromatic rings are linked to each other. Preferred is a substituted or unsubstituted aromatic hydrocarbon group having 6 to 18 carbon atoms, or a substituted or unsubstituted linked aromatic group in which two to five of these aromatic rings are linked to each other. More preferred is a substituted or unsubstituted phenyl group, or a substituted or unsubstituted linked aromatic group in which two to three phenyl groups are linked to each other. Herein, aromatic hydrocarbon groups or aromatic heterocyclic groups in the case of these aromatic rings linked are the same as or different from each other.

Ar⁷ and Ar⁸ are the same as Ar⁵ and Ar⁶ except that no hydrogen is contained. Preferred is a substituted or unsubstituted aromatic hydrocarbon group having 6 to 18 carbon atoms, or a substituted or unsubstituted linked aromatic group in which two to five of these aromatic rings are linked to each other. More preferred is a substituted or unsubstituted phenyl group or a substituted or unsubstituted linked aromatic group in which two to three phenyl groups are linked to each other. Herein, aromatic hydrocarbon groups or aromatic heterocyclic groups in the case of these aromatic rings linked are the same as or different from each other.

Specific examples of the unsubstituted aromatic hydrocarbon group having 6 to 30 carbon atoms, the unsubstituted aromatic heterocyclic group having 3 to 17 carbon atoms, or the linked aromatic group in which two to five of these aromatic rings are linked to each other include a group generated by removing one hydrogen from benzene, naphthalene, acenaphthene, acenaphthylene, azulene, anthracene, chrysene, pyrene, phenanthrene, fluorene, pyridine, pyrimidine, triazine, thiophene, isothiazole, thiazole, pyridazine, pyrrole, pyrazole, imidazole, triazole, thiadiazole, pyrazine, furan, isoxazole, quinoline, isoquinoline, quinoxaline, quinazoline, benzoquinazoline, thiadiazole, phthalazine, tetrazole, indole, benzofuran, benzothiophene, benzoxazole, benzothiazole, indazole, benzimidazole, benzotriazole, benzisothiazole, benzothiadiazole, purine, pyranone, coumarin, isocoumarin, chromone, dibenzofuran, dibenzothiophene, dibenzoselenophene, carbazole, 9,9'-spirobifluorene, or compounds in which two to five of these are linked to each other. Preferred examples thereof include a group generated from benzene, naphthalene, acenaphthene, acenaphthylene, azulene, anthracene, chrysene, pyrene, phenanthrene, fluorene, or compounds in which two to five of these are linked to each other. More preferred is a phenyl group, a biphenyl group, or a terphenyl group. The terphenyl group may be linked linearly or branched.

In the general formula (3), the unsubstituted phenylene group, aromatic hydrocarbon group, aromatic heterocyclic group or linked aromatic group may each have a substituent. In the case of having a substituent, the substituent is preferably deuterium, halogen, a cyano group, an alkyl group having 1 to 10 carbon atoms, a triarylsilyl group having 9 to 30 carbon atoms, an alkenyl group having 2 to 5 carbon atoms, an alkoxy group having 1 to 5 carbon atoms, or a diarylamino group having 12 to 44 carbon atoms.

Note that the number of substituents is 0 to 5 and preferably 0 to 2. When an aromatic hydrocarbon group, an aromatic heterocyclic group, or a linked aromatic group has a substituent, the calculation of the number of carbon atoms does not include the number of carbon atoms of the substituent. However, it is preferred that the total number of carbon atoms including the number of carbon atoms of substituents satisfy the above range.

Specific examples of the substituent include deuterium, cyano, bromo, fluorine, methyl, ethyl, propyl, i-propyl, butyl, t-butyl, pentyl, cyclopentyl, hexyl, cyclohexyl, heptyl, octyl, nonyl, decyl, triphenylsilyl, vinyl, propenyl, butenyl, pentenyl, methoxy, ethoxy, propoxy, butoxy, pentoxy, diphenylamino, naphthylphenylamino, dinaphthylamino, dianthranylamino, diphenanthrenylamino, and dipyrenylamino. Preferred examples thereof include deuterium, cyano, methyl, ethyl, propyl, butyl, pentyl, hexyl, heptyl, octyl, vinyl, propenyl, butenyl, pentenyl, methoxy, ethoxy, propoxy, butoxy, and pentoxy.

L³ represents a direct bond or a substituted or unsubstituted aromatic hydrocarbon group having 6 to 18 carbon atoms. Specific examples of the unsubstituted aromatic hydrocarbon group having 6 to 18 carbon atoms include benzene, naphthalene, acenaphthene, acenaphthylene, azulene, anthracene, chrysene, pyrene, phenanthrene, and fluorene. Preferred is a direct bond or a substituted or unsubstituted phenylene group.

Preferred aspects of the general formula (3) are general formulas (3b) to (3g), more preferably general formulas (3b) to (3d): 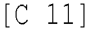 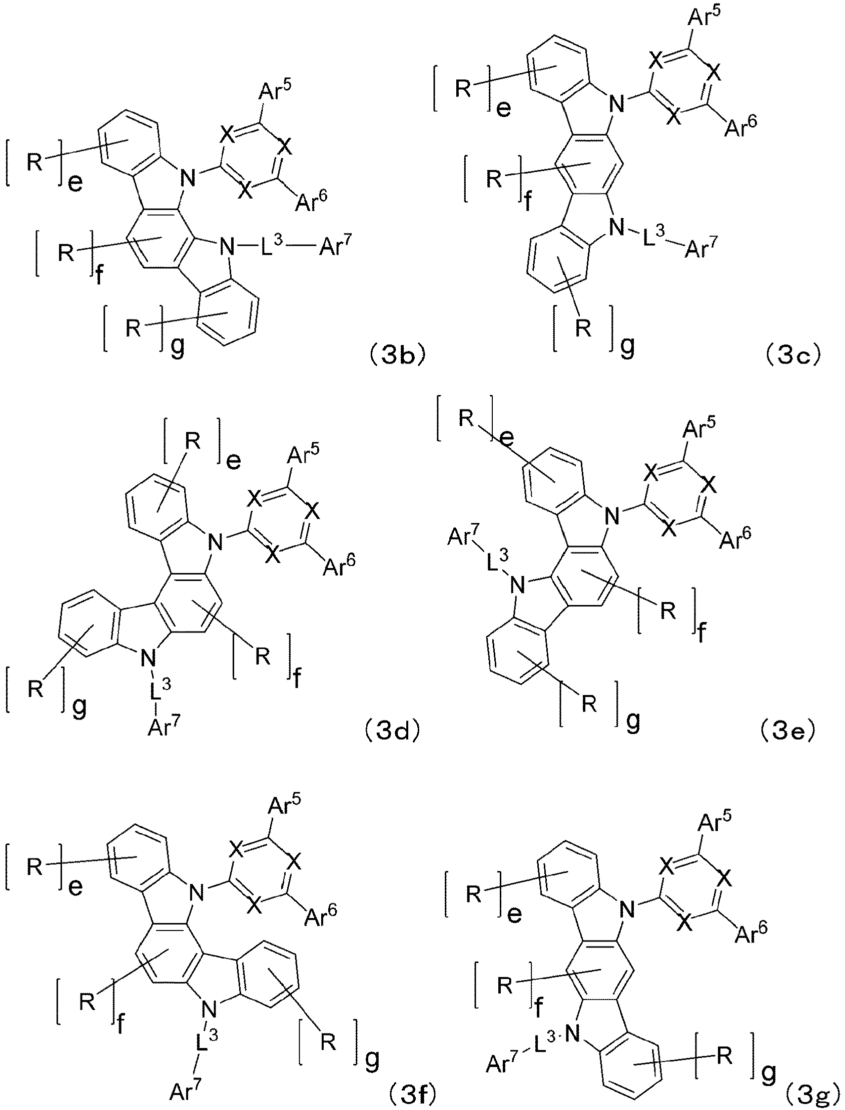 wherein Ar⁵, Ar⁶, Ar⁷, L³, X, R, e, f, and g have the same meaning as in the general formula (3).

Specific examples of the compound represented by the general formula (3) are shown below, but are not limited to these exemplified compounds.

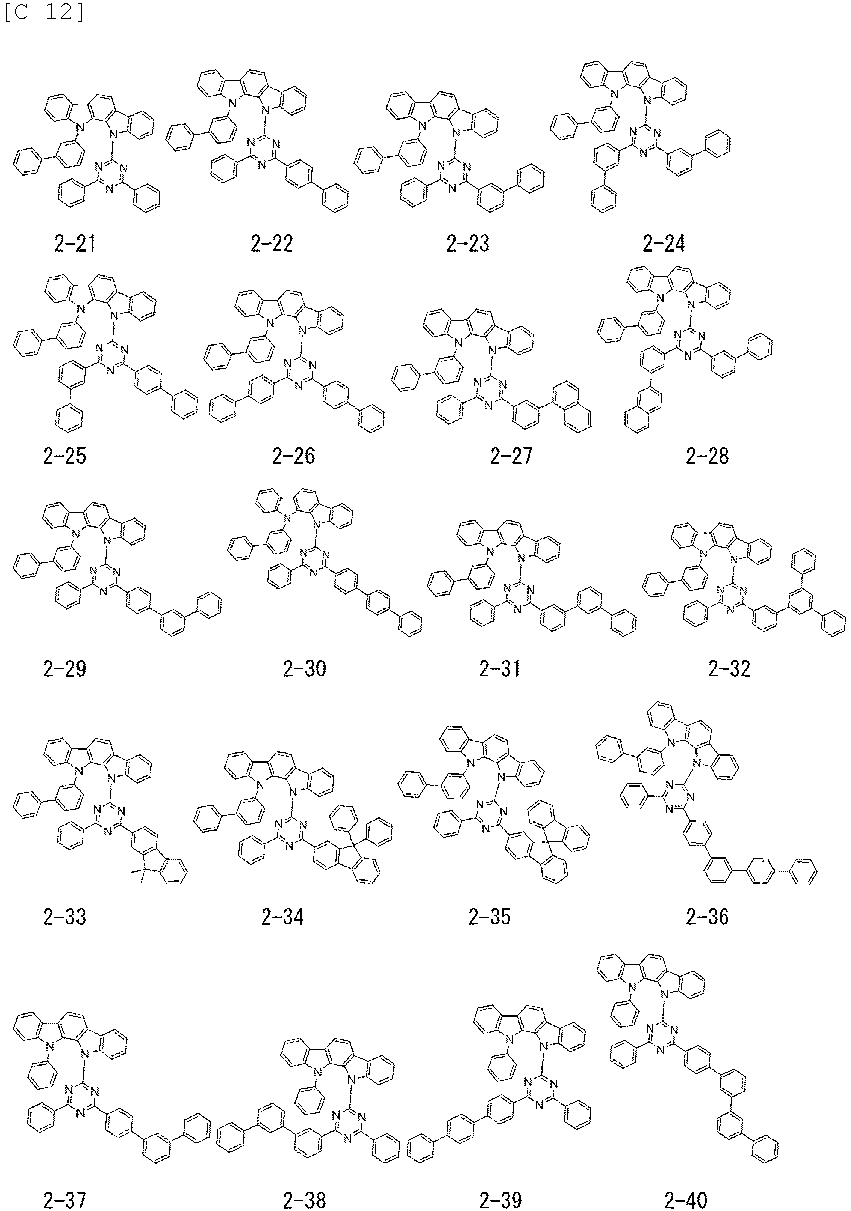

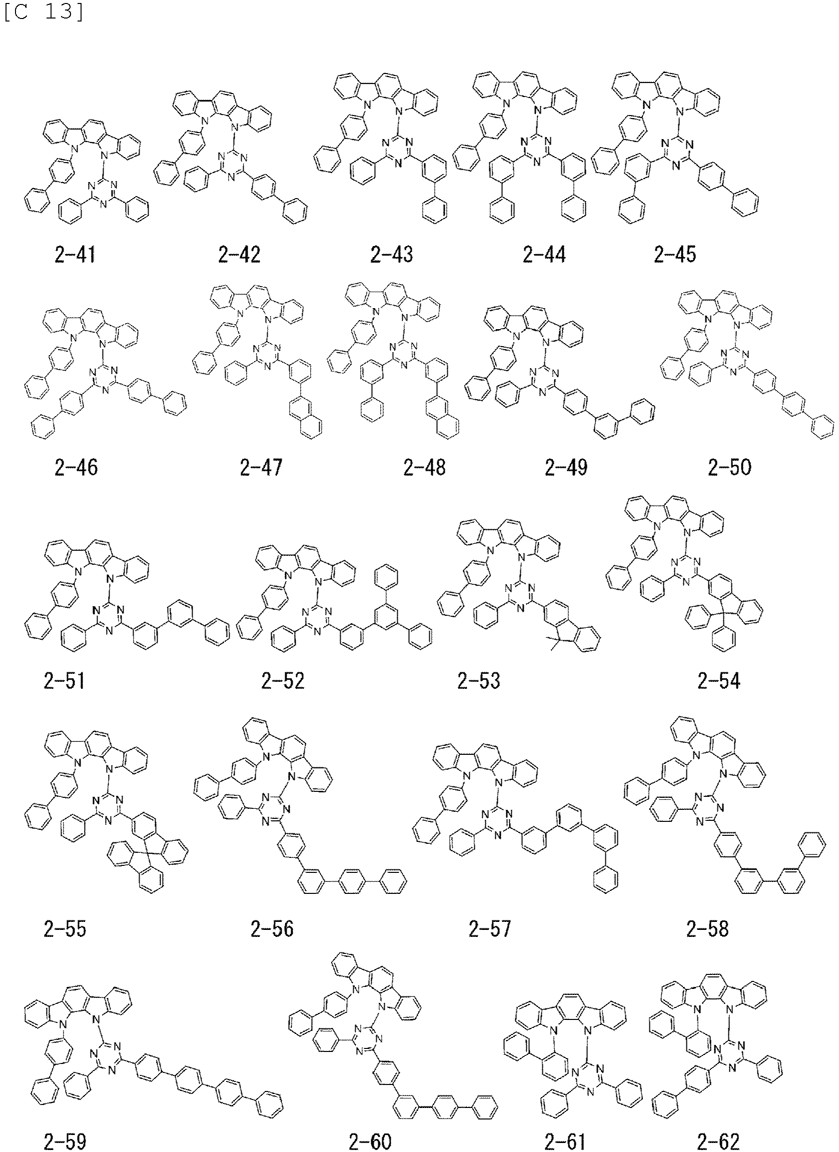

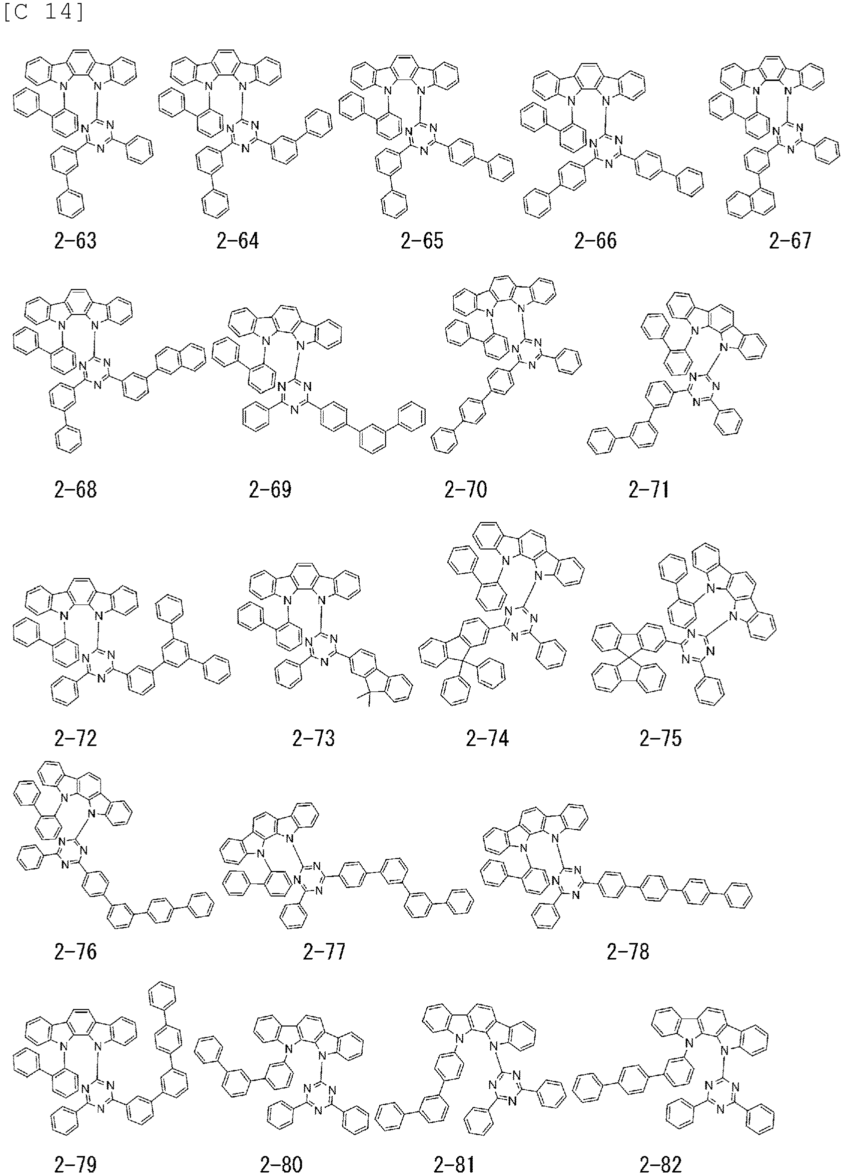

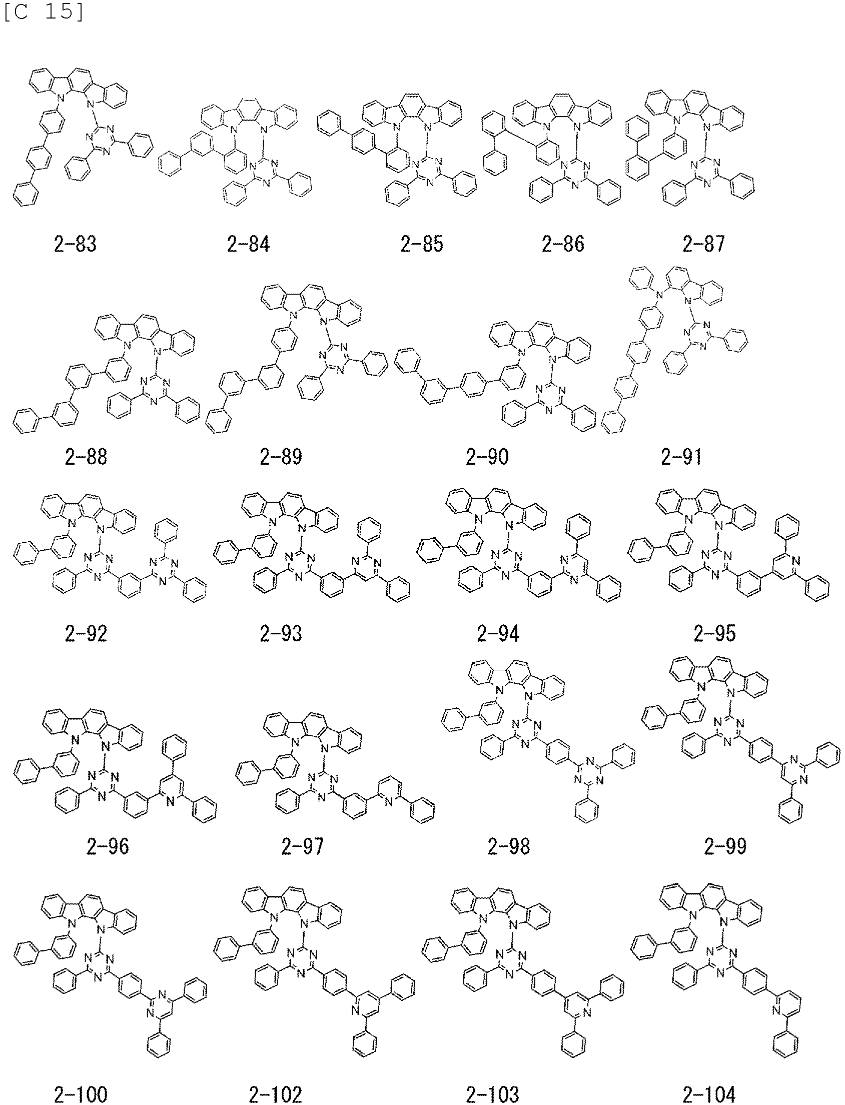

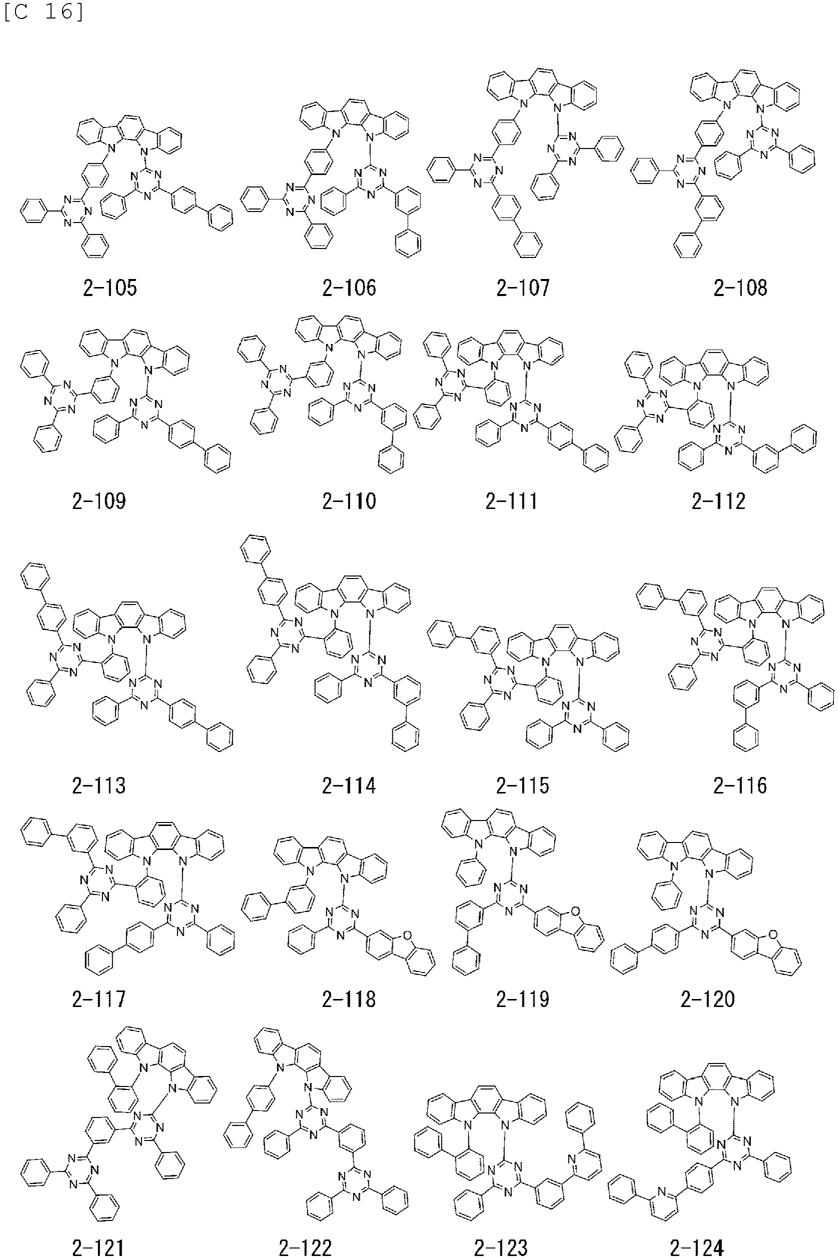

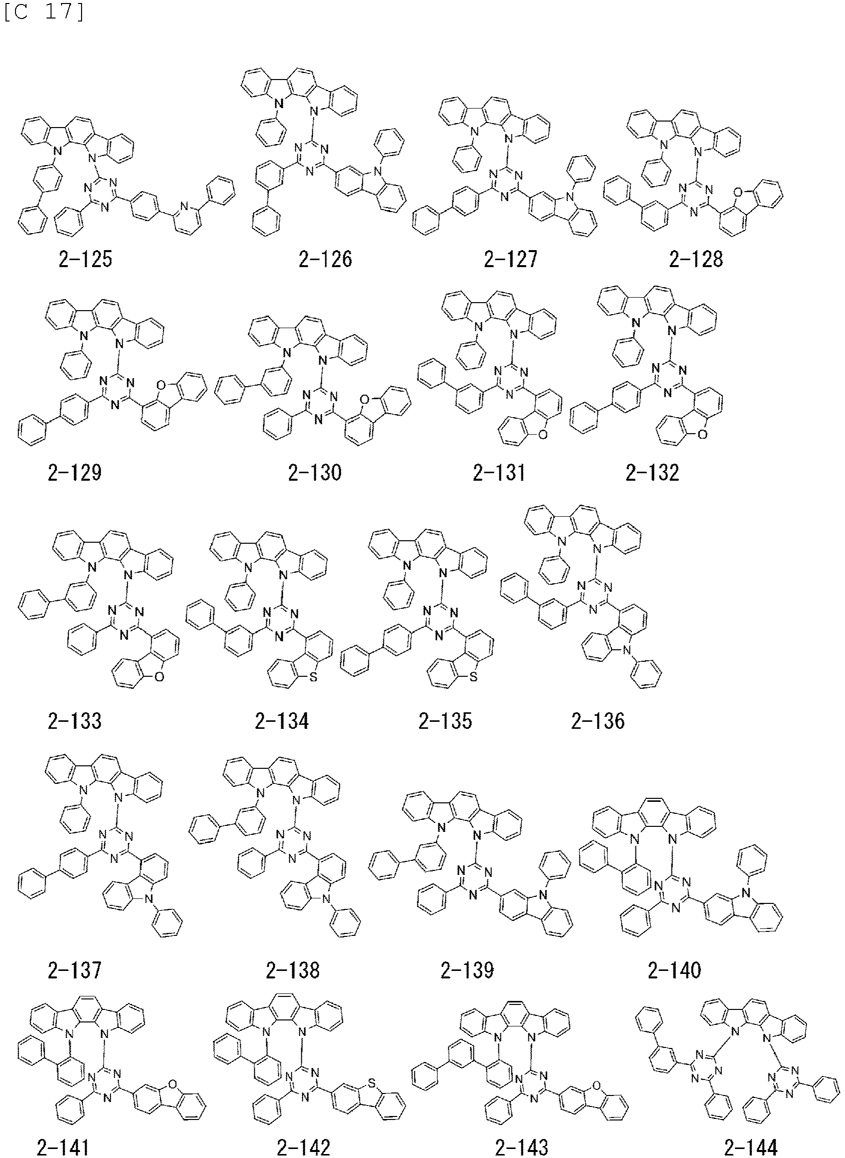

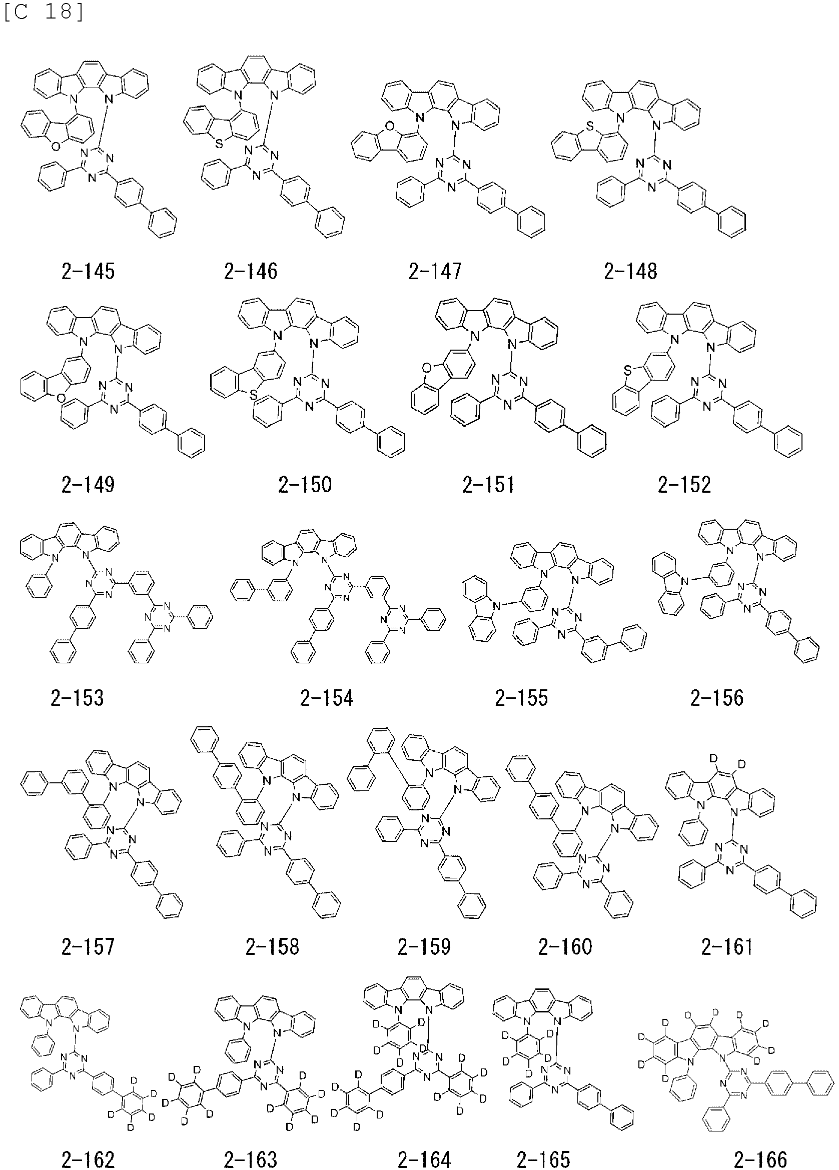

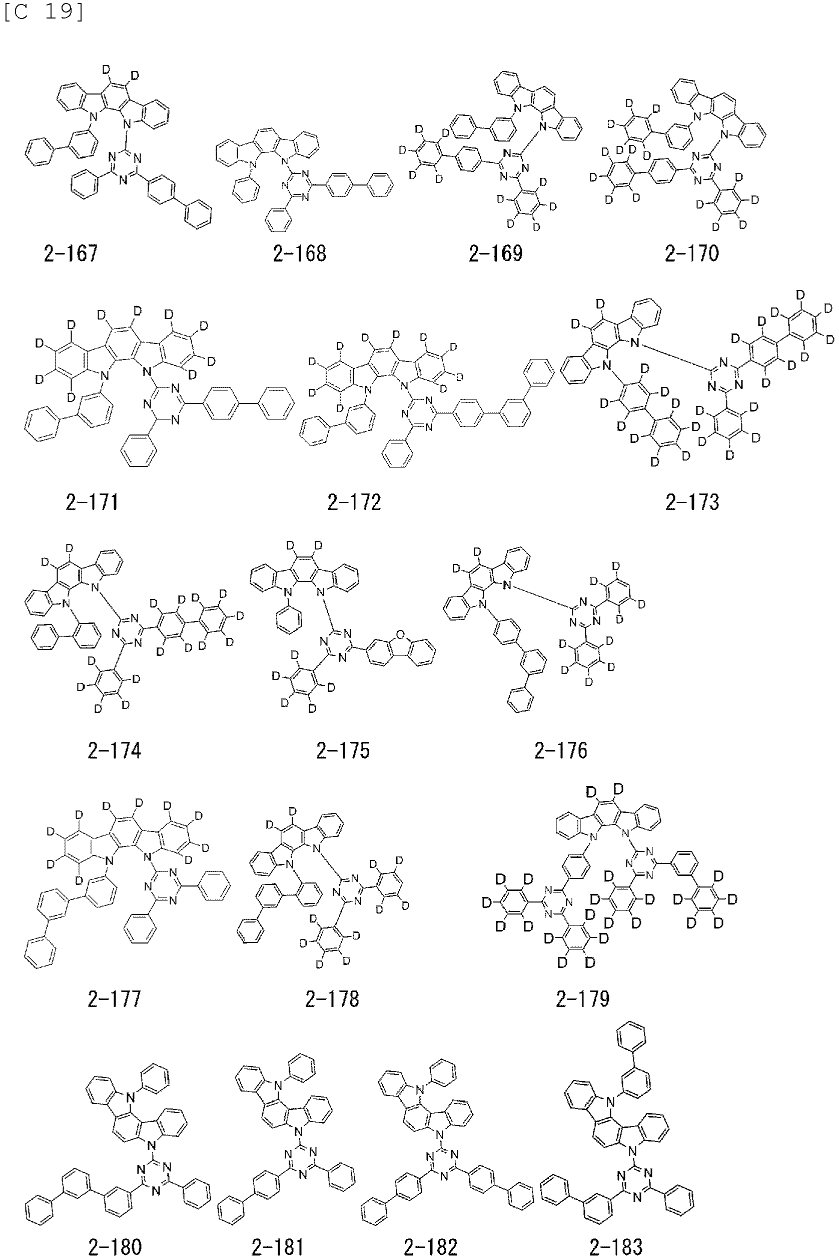

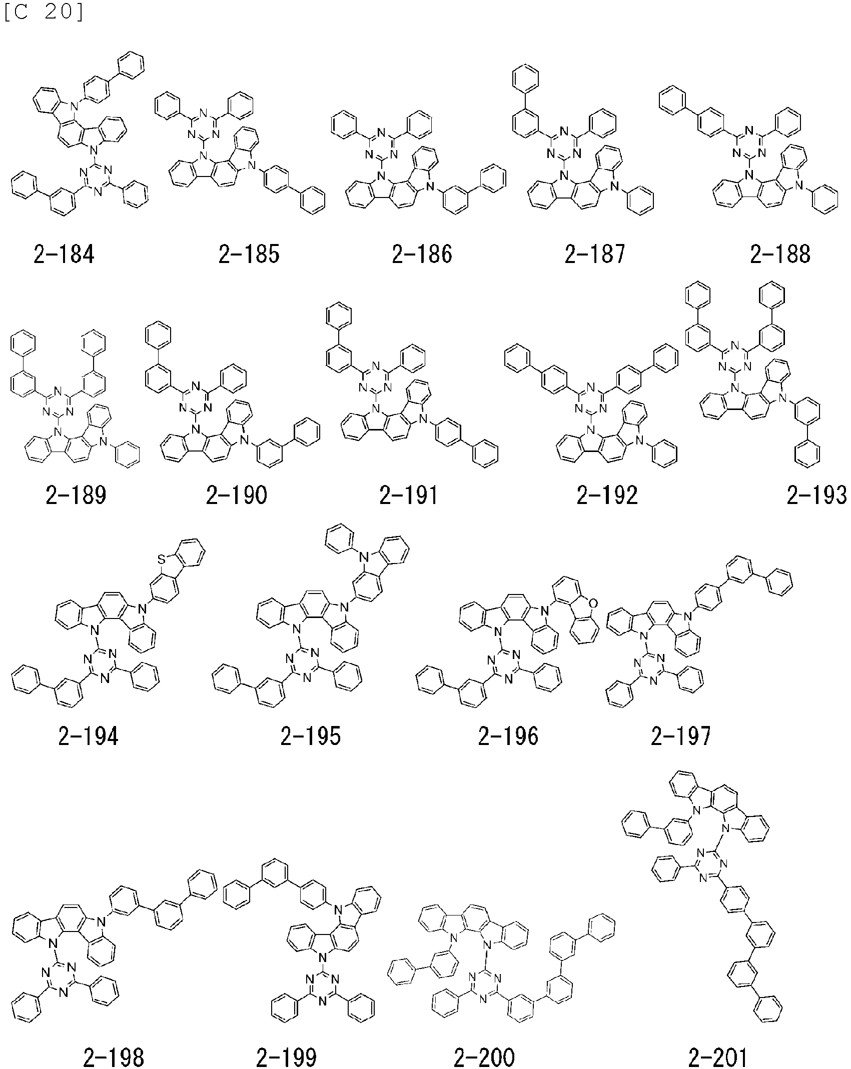

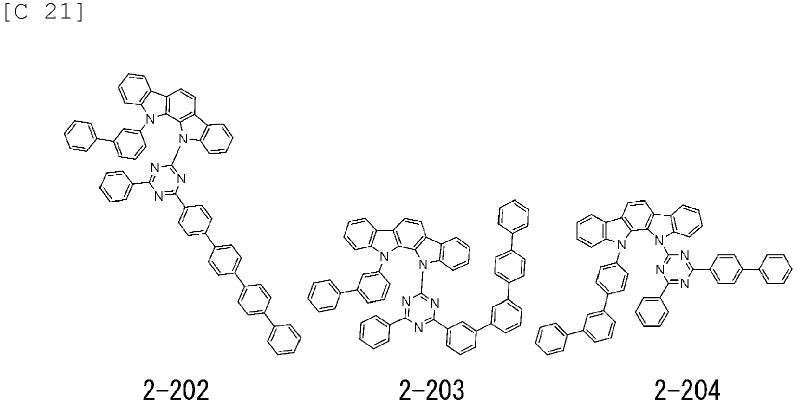

The mixture of the present invention comprises the deuteride of a compound represented by the general formula (1) in which the rate of deuteration of hydrogen atoms on two carbazole rings in the compound is 30% or more, and the compound represented by the general formula (3). The difference in 50% weight reduction temperatures of the deuteride and the compound represented by the general formula (3) is preferably within 20°C.

In addition, regarding the mixing ratio (weight ratio) between the deuteride and the compound represented by the general formula (3) in the mixture of the present invention, the proportion of the compound represented by the general formula (3) may be 20 to 70 wt%, and is preferably 20 to 60 wt%.

The deuteride of a compound represented by the general formula (1) in which the rate of deuteration of hydrogen atoms on two carbazole rings in the compound is 30% or more, or the mixture of the deuteride and the general formula (3), of the present invention, is included in an organic layer, and the organic layer is preferably selected from the group consisting of a light-emitting layer, a hole injection layer, a hole transport layer, an electron transport layer, an electron injection layer, a hole blocking layer and an electron blocking layer.

Preferred is a light-emitting layer, and the light-emitting layer preferably contains at least one light-emitting dopant.

The material for an organic EL device of the present invention, when included in the light-emitting layer, is desirably included as a host. Advantageously, it is preferable that the deuteride of a compound represented by the general formula (1) in which the rate of deuteration of hydrogen atoms on two carbazole rings in the compound is 30% or more, of the present invention, be included as a first host, and the compound represented by the general formula (3) be included as a second host. It is more preferable that the mixture of the deuteride of a compound represented by the general formula (1) in which the rate of deuteration of hydrogen atoms on two carbazole rings in the compound is 30% or more, and the compound represented by the general formula (3), of the present invention, be included as a host.

When the deuteride and the compound represented by the general formula (3) are used as hosts, these compounds can be used by vapor deposition from different individual vapor deposition sources, but the light-emitting layer is preferably produced by premixing these compounds before vapor deposition to provide a mixture (also referred to as "premixture".) for an organic EL device, and vaporizing the premixture simultaneously from one vapor deposition source. In this case, the premixture may be mixed with a light-emitting dopant material necessary for formation of the light-emitting layer, or another host to be used as necessary. However, when there is a large difference in temperatures to provide desired vapor pressure, vapor deposition may be performed from another vapor deposition source.

The organic EL device of the present invention has a plurality of organic layers between electrodes opposite to each other, and at least one of the organic layers is a light-emitting layer. At least one light-emitting layer contains the first host and the second host, and at least one light-emitting dopant.

Next, the structure of the organic EL device of the present invention will be described by referring to the drawing, but the structure of the organic EL device of the present invention is not limited thereto.

Figure 1 is a cross-sectional view showing a structure example of an organic EL device generally used for the present invention, in which there are indicated a substrate 1, an anode 2, a hole injection layer 3, a hole transport layer 4, a light-emitting layer 5, an electron transport layer 6, and a cathode 7. The organic EL device of the present invention may have an exciton blocking layer adjacent to the light-emitting layer and may have an electron blocking layer between the light-emitting layer and the hole injection layer. The exciton blocking layer can be inserted into either of the anode side and the cathode side of the light-emitting layer and inserted into both sides at the same time. The organic EL device of the present invention has the anode, the light-emitting layer, and the cathode as essential layers, and preferably has a hole injection transport layer and an electron injection transport layer in addition to the essential layers, and further preferably has a hole blocking layer between the light-emitting layer and the electron injection transport layer. Note that the hole injection transport layer refers to either or both of a hole injection layer and a hole transport layer, and the electron injection transport layer refers to either or both of an electron injection layer and an electron transport layer.

A structure reverse to that of Figure 1 is applicable, in which a cathode 7, an electron transport layer 6, a light-emitting layer 5, a hole transport layer 4, and an anode 2 are laminated on a substrate 1 in this order. In this case, layers may be added or omitted as necessary.

### - Substrate -

The organic EL device of the present invention is preferably supported on a substrate. The substrate is not particularly limited, and those conventionally used in organic EL devices may be used, and substrates made of, for example, glass, a transparent plastic, or quartz may be used.

### - Anode -

Regarding an anode material for an organic EL device, it is preferable to use a material of a metal, an alloy, an electrically conductive compound, and a mixture thereof, each having a large work function (4 eV or more). Specific examples of such an electrode material include a metal such as Au, and a conductive transparent material such as CuI, indium tin oxide (ITO), SnO₂, and ZnO. In addition, an amorphous material such as IDIXO (In₂O₃-ZnO), which is capable of forming a transparent conductive film, may be used. Regarding the anode, such an electrode material is used to form a thin film by, for example, a vapor-deposition or sputtering method, and a desired shape pattern may be formed by a photolithographic method; or if the pattern accuracy is not particularly required (about 100 µm or more), a pattern may be formed via a desired shape mask when the electrode material is vapor-deposited or sputtered. Alternatively, when a coatable substance such as an organic conductive compound is used, a wet film formation method such as a printing method or a coating method may be used. For taking emitted light from the anode, it is desired to have a transmittance of more than 10%, and the sheet resistance for the anode is preferably several hundreds Ω/□ or less. The film thickness is selected usually within 10 to 1000 nm, preferably within 10 to 200 nm though depending on the material.

### - Cathode -

Meanwhile, regarding a cathode material, preferable to a material of a metal (an electron injection metal), an alloy, an electrically conductive compound, or a mixture thereof, each having a small work function (4 eV or less) are used. Specific examples of such an electrode material include sodium, a sodium-potassium alloy, magnesium, lithium, a magnesium/copper mixture, a magnesium/silver mixture, a magnesium/aluminum mixture, a magnesium/indium mixture, an aluminum/aluminum oxide (Al₂O₃) mixture, indium, a lithium/aluminum mixture, and a rare earth metal. Among these, from the viewpoint of the electron injectability and the durability against oxidation and the like, a mixture of an electron injection metal and a second metal which is a stable metal having a larger work function value is suitable, and examples thereof include a magnesium/silver mixture, a magnesium/aluminum mixture, a magnesium/indium mixture, an aluminum/aluminum oxide mixture, a lithium/aluminum mixture and aluminum. The cathode can be produced by forming a thin film by a method such as vapor-depositing or sputtering of such a cathode material. In addition, the sheet resistance of cathode is preferably several hundreds Ω/□ or less. The film thickness is selected usually within 10 nm to 5 µm, preferably within 50 to 200 nm. Note that for transmission of emitted light, if either one of the anode and cathode of the organic EL device is transparent or translucent, emission luminance is improved, which is convenient.

In addition, formation of a film of the above metal with a thickness of 1 to 20 nm on a cathode, followed by formation of a conductive transparent material described in the description on the anode thereon, enables production of a transparent or translucent cathode, and application of this enables production of a device wherein an anode and a cathode both have transmittance.

### - Light-emitting layer -

The light-emitting layer is a layer that emits light after excitons are generated when holes and electrons injected from the anode and the cathode, respectively, are recombined. As a light-emitting layer, a light-emitting dopant material and a host are contained.

The first host and the second host are preferably used as hosts.

Regarding the first host represented by the general formula (1), one kind of compound may be used, or two or more different compounds may be used. Similarly, regarding the second host represented by the general formula (3), one kind of compound may be used, or two or more different compounds may be used.

If necessary, one, or two or more other known host materials may be used in combination; however, it is preferable that an amount thereof to be used be 50 wt% or less, preferably 25 wt% or less based on the host materials in total.

A preferred method as the method for producing the organic EL device of the present invention is a method comprising providing a premixture comprising the first host and the second host and producing a light-emitting layer by use of the premixture. Additionally, a more preferred method comprises vapor-depositing the premixture by vaporization from a single vapor deposition source. Herein, the premixture is suitably a uniform composition.

When the first host and the second host are premixed and used, it is desirable that a difference in 50% weight reduction temperature (T₅₀) be small in order to produce an organic EL device having favorable characteristics with high reproducibility. The 50% weight reduction temperature is a temperature at which the weight is reduced by 50% when the temperature is raised to 550°C from room temperature at a rate of 10°C/min in TG-DTA measurement under a nitrogen stream reduced pressure (1 Pa). It is considered that vaporization due to evaporation or sublimation the most vigorously occurs around this temperature.

The difference in 50% weight reduction temperatures of the first host and the second host in the premixture is preferably within 20°C. The premixture can be vaporized from a single vapor deposition source and vapor-deposited to thereby obtain a uniform vapor-deposited film. In this case, the premixture may be mixed with a light-emitting dopant material necessary for formation of a light-emitting layer, or another host to be used as necessary. However, when there is a large difference in temperatures to provide desired vapor pressure, vapor-deposition may be performed from another vapor deposition source.

In addition, regarding the mixing ratio (weight ratio) between the first host and the second host, the proportion of the second host may be 20 to 70 wt%, and is preferably 20 to 60 wt% based on the first host and the second host in total.

The premixing method is desirably a method that can allow for mixing as uniformly as possible, and examples thereof include pulverization and mixing, a heating and melting method under reduced pressure or under an atmosphere of an inert gas such as nitrogen, and sublimation, but not limited thereto.

The host and the premixture thereof may be in powder, stick, or granule form.

In the case of use of a plurality of kinds of hosts, such respective hosts can be vapor-deposited from different vapor deposition sources or can be simultaneously vapor-deposited from one vapor deposition source by premixing the hosts before vapor deposition to provide a premixture.

A production method in which a starting material fully or partially deuterated is used and a production method according to hydrogen/deuterium exchange reaction are known as methods for producing a deuterated compound in which the rate of deuteration of hydrogen atoms on two carbazole rings in the compound represented by the general formula (1) is 30% or more. Such a raw material fully or partially deuterated can be purchased from a supplier of a commercially available product, or can be produced according to known hydrogen/deuterium exchange reaction. Examples of such known hydrogen/deuterium exchange reaction include a method including allowing a non-deuterated substance to act on deuterium gas or an equivalent thereof in the presence of a transition metal catalyst, and a method including treating a non-deuterated substance with a deuterated solvent (deuterated benzene or the like) in the presence of an acid catalyst.

When a phosphorescent dopant is used as a light-emitting dopant material, preferred is a phosphorescent dopant including an organic metal complex containing at least one metal selected from ruthenium, rhodium, palladium, silver, rhenium, osmium, iridium, platinum and gold. Specifically, iridium complexes described in J. Am. Chem. Soc. 2001, 123, 4304, JP2013-530515A, US2016/0049599A, US2017/0069848A, US2018/0282356A, US2019/0036043A, or the like, or platinum complexes described in US2018/0013078A, KR2018-094482A, or the like are preferably used, but the phosphorescent dopant is not limited thereto.

Regarding the phosphorescent dopant material, only one kind thereof may be contained in the light-emitting layer, or two or more kinds thereof may be contained. A content of the phosphorescent dopant material is preferably 0.1 to 30 wt% and more preferably 1 to 20 wt% with respect to the host material.

The phosphorescent dopant material is not particularly limited, and specific examples thereof include the following.

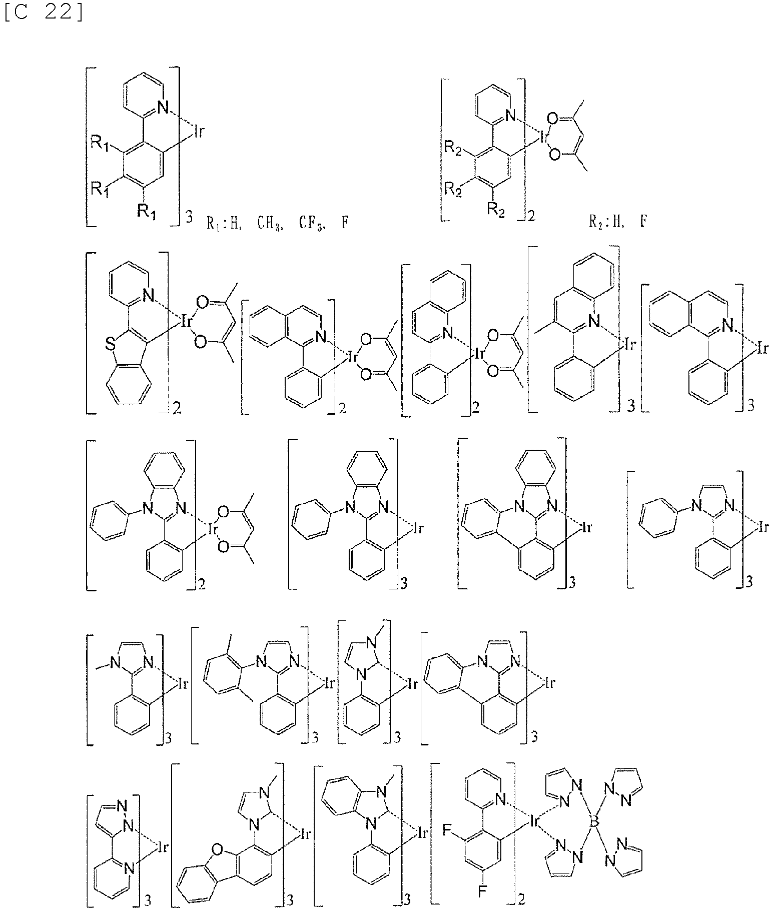

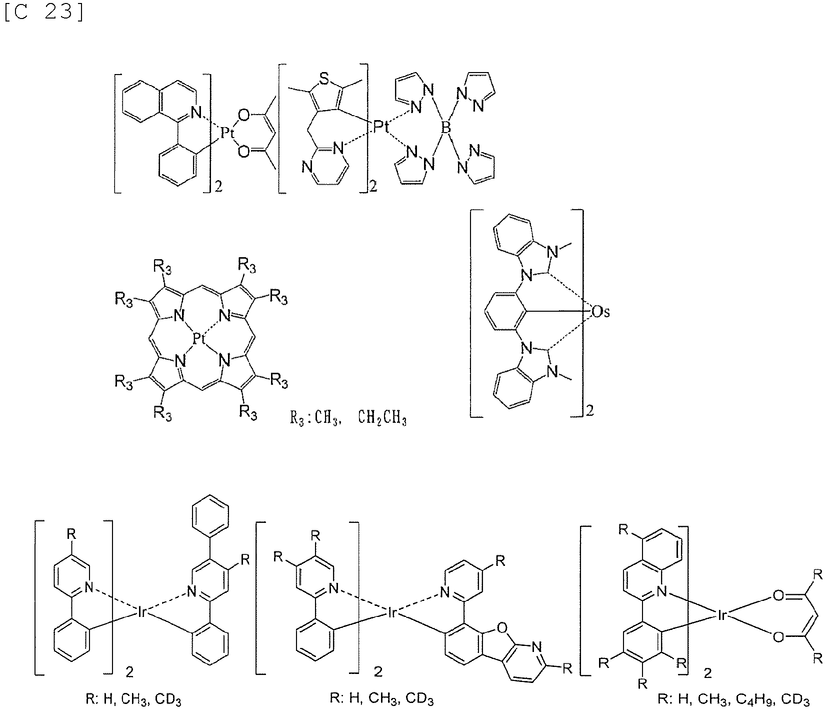

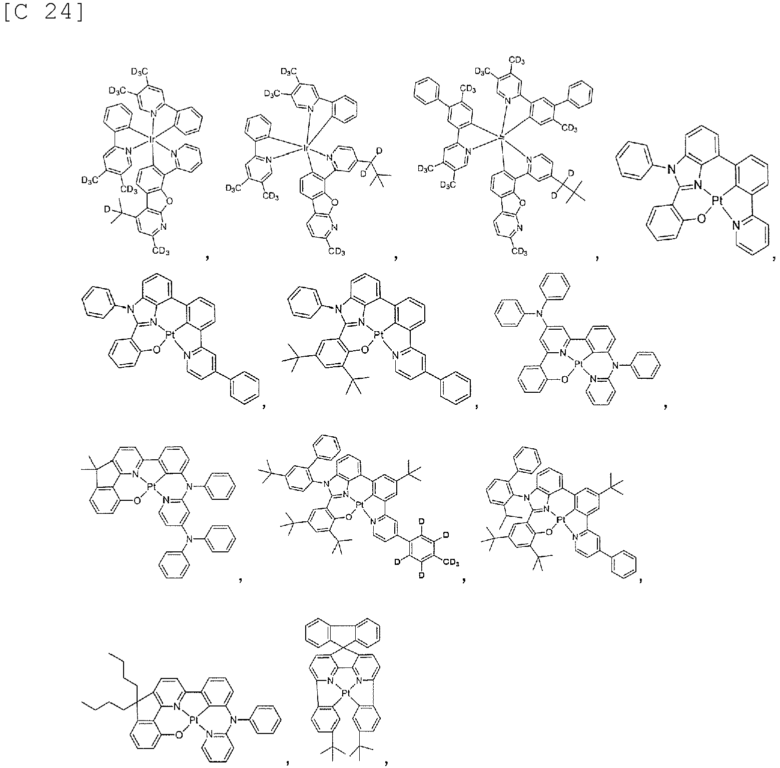

When a fluorescence-emitting dopant is used as the light-emitting dopant material, the fluorescence-emitting dopant is not particularly limited. Examples thereof include benzoxazole derivatives, benzothiazole derivatives, benzimidazole derivatives, styrylbenzene derivatives, polyphenyl derivatives, diphenylbutadiene derivatives, tetraphenyl butadiene derivatives, naphthalimido derivatives, coumarin derivatives, fused aromatic compounds, perinone derivatives, oxadiazole derivatives, oxazine derivatives, aldazine derivatives, pyrrolidine derivatives, cyclopentadiene derivatives, bisstyryl anthracene derivatives, quinacridone derivatives, pyrrolopyridine derivatives, thiadiazolopyridine derivatives, styrylamine derivatives, diketopyrrolopyrrole derivatives, aromatic dimethylidine compounds, metal complexes of 8-quinolinol derivatives or metal complexes of pyromethene derivatives, rare earth complexes, various metal complexes represented by transition metal complexes, polymer compounds such as polythiophene, polyphenylene, and polyphenylene vinylene, and organosilane derivatives. Preferred examples thereof include fused aromatic derivatives, styryl derivatives, diketopyrrolopyrrole derivatives, oxazine derivatives, pyromethene metal complexes, transition metal complexes, and lanthanoid complexes. More preferable examples thereof include naphthalene, pyrene, chrysene, triphenylene, benzo[c]phenanthrene, benzo[a]anthracene, pentacene, perylene, fluoranthene, acenaphthofluoranthene, dibenzo[a,j]anthracene, dibenzo[a,h]anthracene, benzo[a]naphthalene, hexacene, naphtho[2,1-f]isoquinoline, α-naphthaphenanthridine, phenanthrooxazole, quinolino[6,5-f]quinoline, and benzothiophanthrene. These may have an alkyl group, an aryl group, an aromatic heterocyclic group, or a diarylamino group as a substituent.

When a thermally activated delayed fluorescence-emitting dopant is used as the light-emitting dopant material, the thermally activated delayed fluorescence-emitting dopant is not particularly limited. Examples thereof include: metal complexes such as a tin complex and a copper complex; indolocarbazole derivatives described in WO2011/070963A; cyanobenzene derivatives and carbazole derivatives described in Nature 2012, 492, 234; and phenazine derivatives, oxadiazole derivatives, triazole derivatives, sulfone derivatives, phenoxazine derivatives, and acridine derivatives described in Nature Photonics 2014, 8,326.

The thermally activated delayed fluorescence-emitting dopant material is not particularly limited, and specific examples thereof include the following.

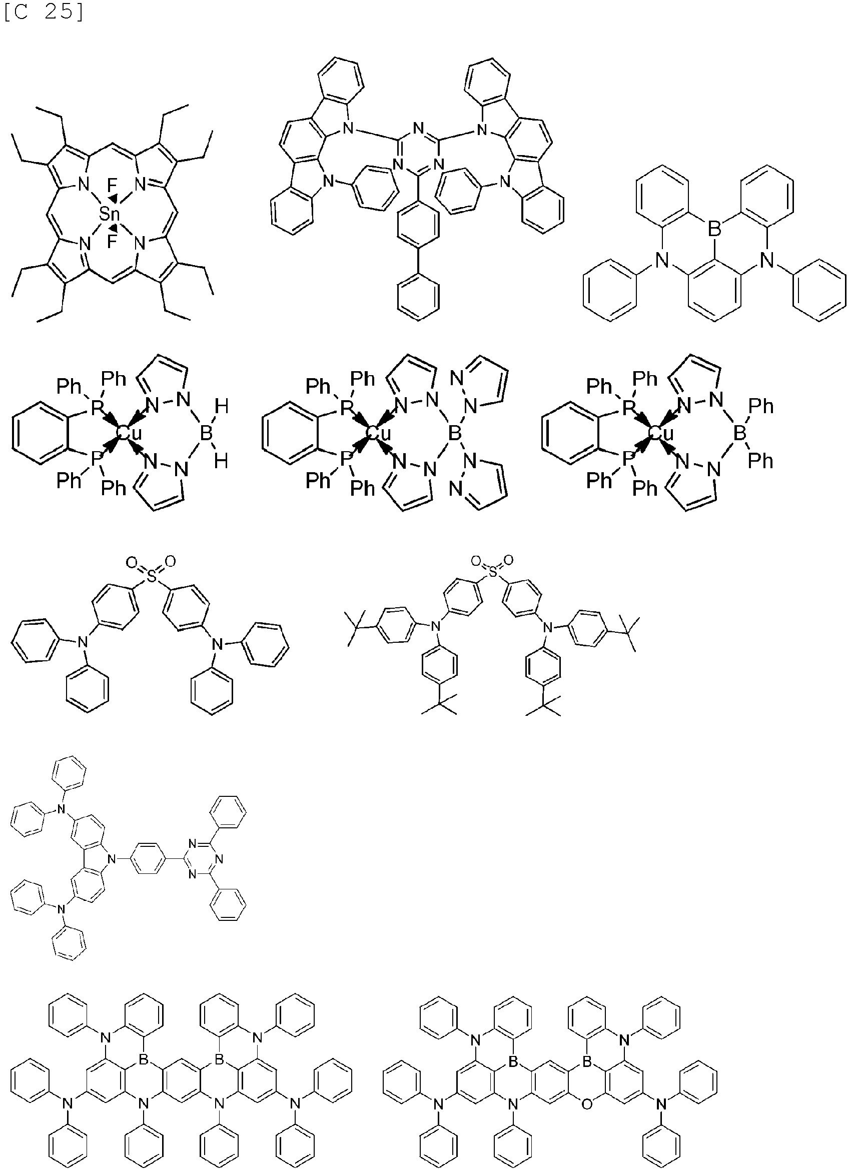

Regarding the thermally activated delayed fluorescence-emitting dopant material, only one kind thereof may be contained in the light-emitting layer, or two or more kinds thereof may be contained. In addition, the thermally activated delayed fluorescence-emitting dopant may be used by mixing with a phosphorescent dopant and a fluorescence-emitting dopant. A content of the thermally activated delayed fluorescence-emitting dopant material is preferably 0.1 wt% to 50 wt% and more preferably 1 wt% to 30 wt% with respect to the host material.

### - Injection Layer -

The injection layer is a layer that is provided between an electrode and an organic layer in order to lower a driving voltage and improve emission luminance, and includes a hole injection layer and an electron injection layer, and may be present between the anode and the light-emitting layer or the hole transport layer, and between the cathode and the light-emitting layer or the electron transport layer. The injection layer can be provided as necessary.

### - Hole Blocking Layer -

The hole blocking layer has a function of the electron transport layer in a broad sense, and is made of a hole blocking material having a function of transporting electrons and a significantly low ability to transport holes, and can block holes while transporting electrons, thereby improving a probability of recombining electrons and holes in the light-emitting layer.

### - Electron Blocking Layer -

The electron blocking layer has a function of a hole transport layer in a broad sense and blocks electrons while transporting holes, thereby enabling a probability of recombining electrons and holes in the light-emitting layer to be improved.

Regarding the material of the electron blocking layer, a known electron blocking layer material can be used and a material of the hole transport layer to be described below can be used as necessary. A film thickness of the electron blocking layer is preferably 3 to 100 nm, and more preferably 5 to 30 nm.

### - Exciton Blocking Layer -

The exciton blocking layer is a layer for preventing excitons generated by recombination of holes and electrons in the light-emitting layer from being diffused in a charge transport layer, and insertion of this layer allows excitons to be efficiently confined in the light-emitting layer, enabling the luminous efficiency of the device to be improved. The exciton blocking layer can be inserted, in a device having two or more light-emitting layers adjacent to each other, between two adjacent light-emitting layers.

Regarding the material of the exciton blocking layer, a known exciton blocking layer material can be used. Examples thereof include 1,3-dicarbazolyl benzene (mCP) and bis(8-hydroxy-2-methylquinoline)-(4-phenylphenoxy)aluminum (III) (BAlq).

### - Hole Transport Layer -

The hole transport layer is made of a hole transport material having a function of transporting holes, and the hole transport layer can be provided as a single layer or a plurality of layers.

The hole transport material has either hole injection, transport properties or electron barrier properties, and may be an organic material or an inorganic material. For the hole transport layer, any one selected from conventionally known compounds can be used. Examples of such a hole transport material include porphyrin derivatives, arylamine derivatives, triazole derivatives, oxadiazole derivatives, imidazole derivatives, polyarylalkane derivatives, pyrazoline derivatives and pyrazolone derivatives, phenylenediamine derivatives, arylamine derivatives, amino-substituted chalcone derivatives, oxazole derivatives, styryl anthracene derivatives, fluorenone derivatives, hydrazone derivatives, stilbene derivatives, silazane derivatives, an aniline copolymer, and a conductive polymer oligomer, and particularly a thiophene oligomer. Use of porphyrin derivatives, arylamine derivatives, or styrylamine derivatives is preferred. Use of arylamine derivatives is more preferred.

### - Electron Transport Layer -

The electron transport layer is made of a material having a function of transporting electrons, and the electron transport layer can be provided as a single layer or a plurality of layers.

The electron transport material (which may also serve as a hole blocking material) may have a function of transferring electrons injected from the cathode to the light-emitting layer. For the electron transport layer, any one selected from conventionally known compounds can be used, and examples thereof include polycyclic aromatic derivatives such as naphthalene, anthracene, and phenanthroline, tris(8-hydroxyquinoline)aluminum(III) derivatives, phosphine oxide derivatives, nitrosubstituted fluorene derivatives, diphenylquinone derivatives, thiopyrandioxide derivatives, carbodiimide, fluorenylidene methane derivatives, anthraquinodimethane and anthrone derivatives, bipyridine derivatives, quinoline derivatives, oxadiazole derivatives, benzimidazole derivatives, benzothiazole derivatives, and indolocarbazole derivatives. In addition, a polymer material in which the above material is introduced into a polymer chain or the above material is used for a main chain of a polymer can be used.

### Examples

Hereafter, the present invention will be described in detail by referring to Examples, but the present invention is not limited to these Examples and can be implemented in various forms without departing from the gist thereof.

### Synthesis Example 1

Compound (2) was synthesized in accordance with the next reaction formula.

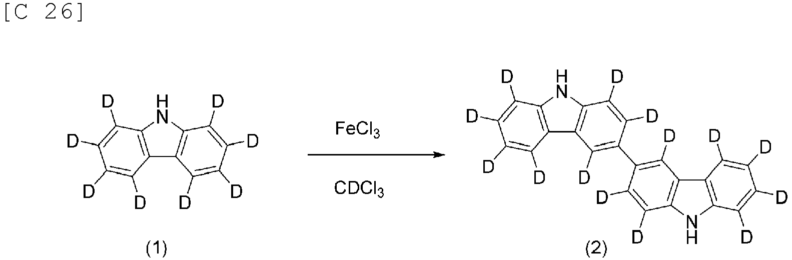

To compound (1) (10.0 g) were added 150 ml of deuterochloroform and 30.5 g of iron (III) chloride, and stirred at room temperature under a nitrogen atmosphere for 12 hours. A reaction liquid was diluted by addition of 300 g of methanol, and separated and purified to give 2.9 g of white solid compound (2) as a deuteride.

### Synthesis Example 2

Compound (3) was synthesized in accordance with the next reaction formula. Herein, compound (2) is shown as an example of a structural formula in which the rate of deuteration of hydrogen on two carbazole rings is 100%, and the same also applies to compound (3).

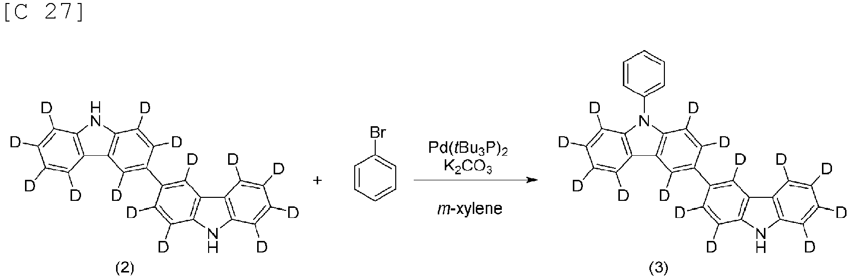

To compound (2) (3.5 g) were added 1.7 g of bromobenzene, 300 ml of m-xylene, 0.3 g of bis(tri-tert-butylphosphine)palladium, and 7.0 g of potassium carbonate, and stirred under heating reflux under a nitrogen atmosphere for 5 hours. A reaction liquid was cooled, and then separated and purified to give 2.0 g of white solid compound (3) as a deuteride.

### Synthesis Example 3

Compound 1-2a was synthesized in accordance with the next reaction formula. Herein, compound 1-2a is shown as an example of a structural formula in which the rate of deuteration of hydrogen on two carbazole rings is 100%.

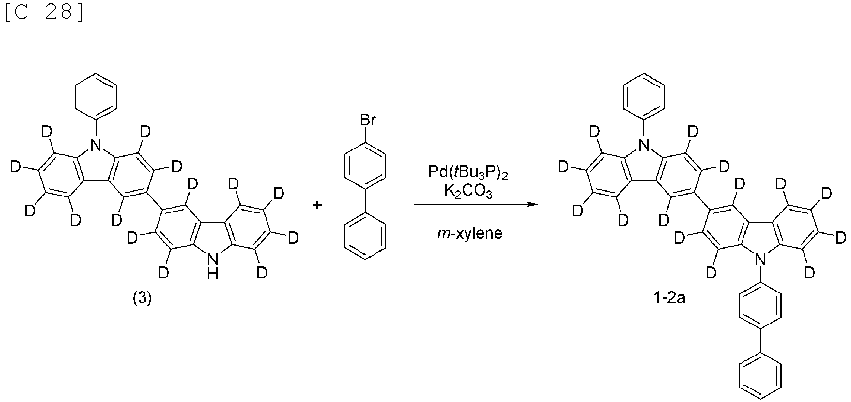

To compound (3) (3.0 g) were added 2.0 g of p-bromobiphenyl, 100 ml of m-xylene, 0.2 g of bis(tri-tert-butylphosphine)palladium, and 4.9 g of potassium carbonate, and stirred under heating reflux under a nitrogen atmosphere for 5 hours. A reaction liquid was cooled, and then separated and purified to give 1.5 g of white solid compound 1-2a as a deuteride.

The rate of deuteration in 1-2a was determined by a proton nuclear magnetic resonance method. A measurement sample was prepared by dissolving 1-2a (5.0 mg) and dimethylsulfone (2.0 mg) as an internal standard material in deuterated tetrahydrofuran (1.0 ml). The average proton concentration [mol/g] of 1-2a included in the measurement sample was calculated from the ratio between the integrated intensities derived from the internal standard material and 1-2a. The average proton concentration [mol/g] of a non-deuterated substance (corresponding to Comparative Example compound A) of 1-2a was also calculated in the same manner. Next, the average rate of deuteration in 1-2a was calculated by calculating the ratio between the proton concentration of 1-2a and the proton concentration of the non-deuterated substance of 1-2a, and subtracting the ratio from 1. Herein, the rate of deuteration in a partial structure was calculated in the same manner, from the integrated intensity with respect to a chemical shift assigned to an objective partial structure.

As a result of the above calculation, the rate of deuteration in 1-2a was 48%, of which the rate of deuteration of hydrogen on two carbazole rings was 96%.

### Synthesis Example 4

Compound 1-2b was synthesized in accordance with the next reaction formula. Dα in the formula represents the average number of substitutions with deuterium in the whole compound 1-2b.

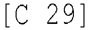

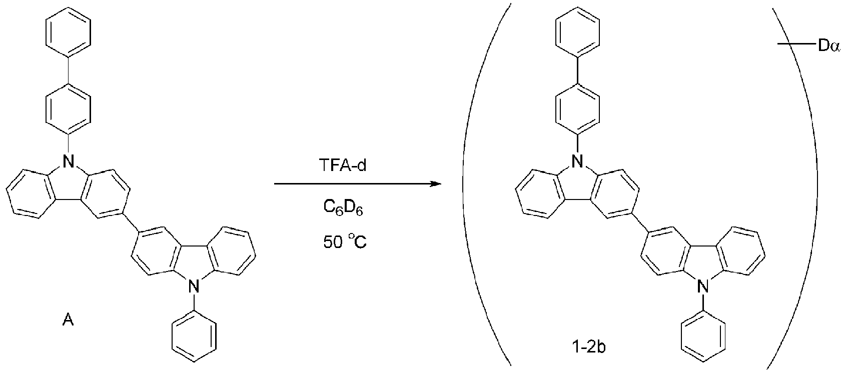

To 8.3 g of comparative compound A were added 160 ml of deuterated benzene (C₆D₆) and 10.0 g of deuterated trifluoroacetic acid (TFA-d), and heated and stirred at 50°C under a nitrogen atmosphere for 6.5 hours. A reaction liquid was added to a deuterium aqueous solution (200 ml) of sodium carbonate (7.4 g) and rapidly cooled, and separated and purified to give 2.8 g of white solid compound 1-2b as a deuteride. The rate of deuteration in 1-2b was calculated in the same manner as in 1-2a, and as a result, the whole rate of deuteration was 25%, of which the rate of deuteration of hydrogen on two carbazole rings was 44%.

### Synthesis Examples 5 to 6

Compounds 1-5 and 1-6 as deuterides were synthesized by reaction performed in the same manner as in Synthesis Examples 1 to 4. Herein, compounds 1-5 and 1-6 are shown as examples of a structural formula in which the rate of deuteration of hydrogen on two carbazole rings is 100%. The rate of deuteration in each of compounds 1-5 and 1-6 was calculated in the same manner as in 1-2a, and as a result, the whole rate of deuteration in each of both compounds 1-5 and 1-6 was 42%, of which the rate of deuteration of hydrogen on two carbazole rings was 96%.

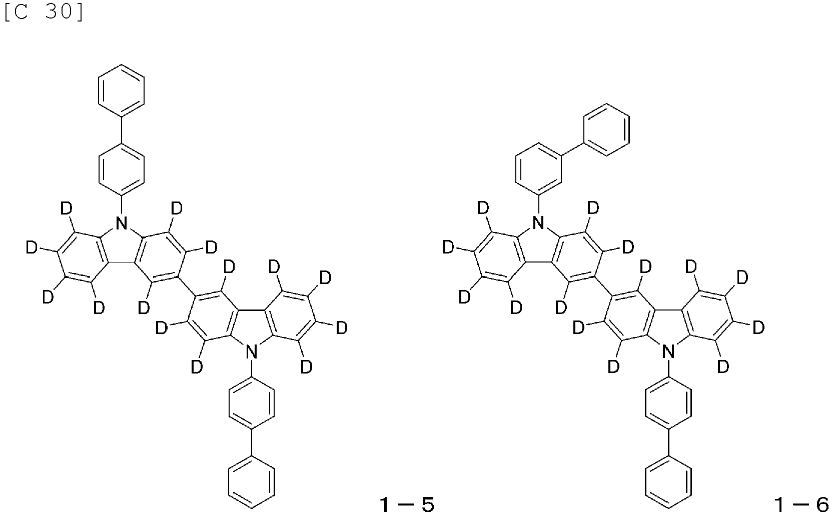

For comparison, Comparative Example compounds A, B, B', C, and E were synthesized (Comparative Synthesis Examples 1 to 5). Dβ in Comparative Example compound B' represents the average number of substitutions with deuterium in the whole compound. The rate of deuteration in each of comparative compounds B and B' was also determined in the same manner as in 1-2a. Herein, calculation was made in terms of the average number of substitutions with deuterium, by multiplying the rate of deuteration with the number of hydrogen atoms before deuteration. Such Comparative Example compounds A, B, B', C, and E are shown as compounds in the last of the description of the present Examples.

**[Table 1]**

| Compound | | Whole | | Carbazole ring | |
|---|---|---|---|---|---|
| | | Rate of deuter ation | Number of hydrogen atoms before deuteration | Rate of deuter ation | Number of hydrogen atoms before deuteration |
| Synthesis Example 3 | 1-2a | 48% | 28 | 96% | 14 |
| Synthesis Example 4 | 1-2b | 25% | 28 | 44% | 14 |
| Synthesis Example 5 | 1-5 | 42% | 32 | 96% | 14 |
| Synthesis Example 6 | 1-6 | 42% | 32 | 96% | 14 |
| Comparativ e Synthesis Example 1 | Comparative Example compound A | 0% | 28 | 0% | 14 |
| Comparativ e Synthesis Example 2 | Comparative Example compound B | 17% | 28 | 0% | 14 |
| Comparativ e Synthesis Example 3 | Comparative Example compound B' | 11% | 28 | 18% | 14 |
| Comparativ e Synthesis Example 4 | Comparative Example compound C | 0% | 32 | 0% | 14 |
| Comparativ e Synthesis Example 5 | Comparative Example compound E | 0% | 32 | 0% | 14 |

### Example 1

On a glass substrate on which an anode made of ITO with a film thickness of 110 nm was formed, respective thin films were laminated by a vacuum evaporation method at a degree of vacuum of 4.0 × 10⁻⁵ Pa. First, HAT-CN was formed with a thickness of 25 nm as a hole injection layer on ITO, and next, Spiro-TPD was formed with a thickness of 30 nm as a hole transport layer. Next, HT-1 was formed with a thickness of 10 nm as an electron blocking layer. Then, compound 1-2a as a first host, compound 2-22 as a second host and Ir(ppy)₃ as a light-emitting dopant were co-vapordeposited from different vapor deposition sources, respectively, to form a light-emitting layer with a thickness of 40 nm. In this case, co-vapor deposition was performed under vapor deposition conditions such that the concentration of Ir(ppy)₃ was 10 wt% and the total concentration of the first host and the second host was 90 wt%, and furthermore the weight ratio between the first host and the second host was 70:30. Next, ET-1 was formed with a thickness of 20 nm as an electron transport layer. Further, LiF was formed with a thickness of 1 nm as an electron injection layer on the electron transport layer. Finally, Al was formed with a thickness of 70 nm as a cathode on the electron injection layer to produce an organic EL device.

### Examples 2 to 12

Organic EL devices were produced in the same manner as in Example 1 except that compounds shown in Table 2 were used as the first host and the second host.

### Comparative Examples 1 to 13

Organic EL devices were produced in the same manner as in Example 1 except that compounds shown in Table 2 were used as the first host and the second host.

Evaluation results of the produced organic EL devices are shown in Table 2. In the table, the luminance, driving voltage, and luminous efficiency are values at a driving current of 20 mA/cm², and they exhibit initial characteristics. LT70 is a time period needed for the initial luminance to be reduced to 70%, and it represents lifetime characteristics. The numbers with which the first host and the second host are marked are numbers with which the exemplified compounds and Synthesis Examples are marked.

**[Table 2]**

| | First host compound | Second host compound | Voltage (V) | Luminanc e (cd/m²) | Power efficiency (lm/W) | LT70 (h) |
|---|---|---|---|---|---|---|
| Example 1 | 1-2a | 2-22 | 5.70 | 11600 | 32.0 | 3040 |
| Example 2 | 1-2b | 2-22 | 5.60 | 12100 | 33.9 | 2600 |
| Example 3 | 1-5 | 2-22 | 5.30 | 12100 | 35.8 | 2470 |
| Example 4 | 1-6 | 2-22 | 5.50 | 12000 | 34.3 | 2420 |
| Example 5 | 1-2a | 2-37 | 5.90 | 12100 | 32.2 | 3050 |
| Example 6 | 1-2b | 2-37 | 5.80 | 11700 | 31.7 | 3230 |
| Example 7 | 1-5 | 2-37 | 5.40 | 11900 | 34.6 | 2630 |
| Example 8 | 1-6 | 2-37 | 5.60 | 11700 | 32.8 | 2570 |
| Example 9 | 1-2a | 2-168 | 5.50 | 12100 | 34.5 | 2560 |
| Example 10 | 1-2b | 2-168 | 5.50 | 11700 | 33.4 | 2650 |
| Example 11 | 1-5 | 2-168 | 5.10 | 11900 | 36.6 | 2330 |
| Example 12 | 1-6 | 2-168 | 5.30 | 11700 | 34.7 | 2330 |
| Comparativ e Example 1 | A | 2-22 | 5.70 | 11800 | 32.5 | 2400 |
| Comparativ e Example 2 | B | 2-22 | 5.70 | 11700 | 32.2 | 2380 |
| Comparativ e Example 3 | B' | 2-22 | 5.70 | 11800 | 32.5 | 2420 |
| Comparativ e Example 4 | C | 2-22 | 5.30 | 11600 | 34.4 | 1750 |
| Comparativ e Example 5 | E | 2-22 | 5.40 | 11700 | 34.0 | 1840 |
| Comparativ e Example 6 | A | 2-37 | 5.80 | 11700 | 31.7 | 2510 |
| Comparativ e Example 7 | B | 2-37 | 5.80 | 11800 | 31.9 | 2490 |
| Comparativ e Example 8 | C | 2-37 | 5.40 | 11700 | 34.0 | 1880 |
| Comparativ e Example 9 | E | 2-37 | 5.50 | 11600 | 33.1 | 1260 |
| Comparativ e Example 10 | A | 2-168 | 5.50 | 11700 | 33.4 | 2300 |
| Comparativ e Example 11 | B | 2-168 | 5.50 | 11900 | 34.0 | 2270 |
| Comparativ e Example 12 | C | 2-168 | 5.10 | 11800 | 36.3 | 1750 |
| Comparativ e Example 13 | E | 2-168 | 5.20 | 11800 | 35.6 | 1450 |

From the results in Table 2, it is understood that Examples 1 to 12 exhibited long lifetime characteristics as compared with Comparative Examples.

### Examples 13 to 16

A premixture was obtained by weighing compounds shown in Table 3, used as a first host and a second host, at a weight ratio shown in Table 3 and mixing them while grinding in a mortar. Organic EL devices were produced in the same manner as in Example 1 except that the premixture was vapor-deposited from one vapor deposition source.

### Comparative Examples 14 to 18

A premixture was obtained by weighing compounds shown in Table 3, used as a first host and a second host, at a weight ratio shown in Table 3 and mixing them while grinding in a mortar. Organic EL devices were produced in the same manner as in Example 1 except that the premixture was vapor-deposited from one vapor deposition source.

Evaluation results of the produced organic EL devices are shown in Table 3. In the table, the luminance, driving voltage, and luminous efficiency are values at a driving current of 20 mA/cm², and they exhibit initial characteristics. LT70 is a time period needed for the initial luminance to be reduced to 70%, and it represents lifetime characteristics. The numbers with which the first host and the second host are marked are numbers with which the exemplified compounds and Synthesis Examples are marked.

**[Table 3]**

| | First host compound | Second host compound | Weight ratio | Voltage (V) | Luminance (cd/m²) | Power efficiency (lm/W) | LT70 (h) |
|---|---|---|---|---|---|---|---|
| Example 13 | 1-2a | 2-22 | 70 : 30 | 5.80 | 11600 | 31.4 | 3100 |
| Example 14 | 1-2a | 2-22 | 60 : 40 | 5.20 | 11400 | 34.4 | 2320 |
| Example 15 | 1-2a | 2-22 | 80 : 20 | 5.50 | 10400 | 29.7 | 1150 |
| Example 16 | 1-2b | 2-22 | 70 : 30 | 5.70 | 12500 | 34.4 | 2690 |
| Comparative Example 14 | A | 2-22 | 70 : 30 | 5.70 | 11700 | 32.2 | 2390 |
| Comparative Example 15 | A | 2-22 | 60 : 40 | 5.10 | 11900 | 36.6 | 1690 |
| Comparative Example 16 | A | 2-22 | 80 : 20 | 6.10 | 10700 | 27.5 | 1540 |
| Comparative Example 17 | B | 2-22 | 70 : 30 | 5.70 | 11800 | 32.5 | 2380 |
| Comparative Example 18 | B' | 2-22 | 70 : 30 | 5.70 | 11800 | 32.5 | 2430 |

From the results in Table 3, it is understood that Examples 13 to 16 exhibited high power efficiency and long lifetime characteristics as compared with Comparative Examples.

Table 4 shows the 50% weight reduction temperatures (T₅₀) of compounds 1-2a, 1-2b and 2-22.

**[Table 4]**

| Compound | T₅₀[°C] |
|---|---|
| 1-2a | 280 |
| 1-2b | 279 |
| 2-22 | 278 |
| A | 279 |
| B | 280 |
| B' | 280 |

Compounds used in Examples and Comparative Examples are shown below.

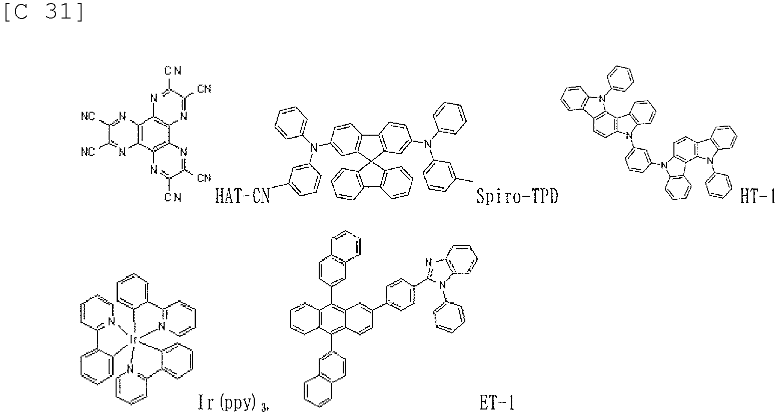

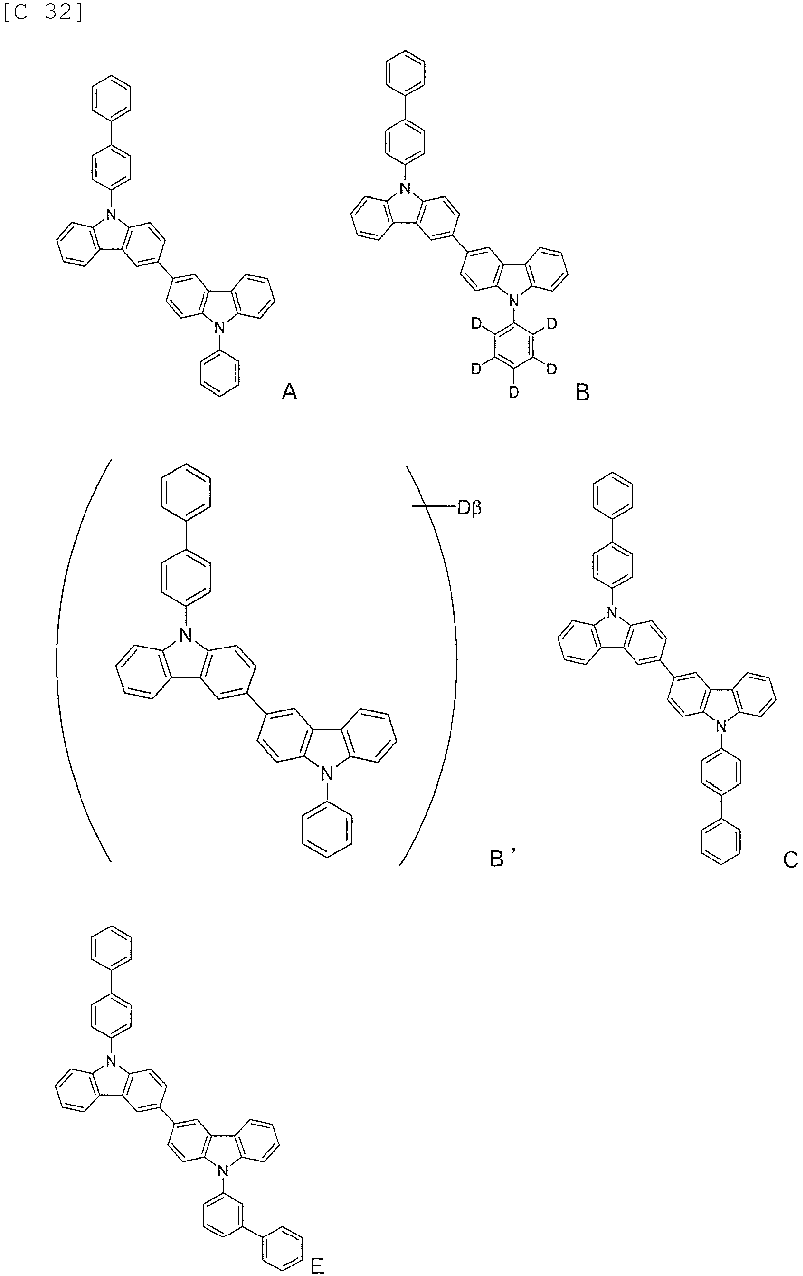

### Industrial Applicability

The deuteride of the present invention can be used as a material for an organic EL device, has a structure represented by the general formula (1), and has deuterium on a carbazole ring, and therefore it is supposed that the number of substitutions and the substitution position(s) can be appropriately designed to thereby allow for enhancements in stabilities of an excited state and an ionic state and control of charge injection/transport properties at a high level. An organic EL device in which the deuteride is used is thus considered to have a long lifetime and a low voltage, and exhibit characteristics at a practical use level.

### Reference Signs List

1 substrate, 2 anode, 3 hole injection layer, 4 hole transport layer, 5 light-emitting layer, 6 electron transport layer, 7 cathode.

## Claims

1. A deuteride of a compound represented by the following general formula (1), wherein a rate of deuteration of hydrogen atoms on two carbazole rings in the compound is 30% or more: 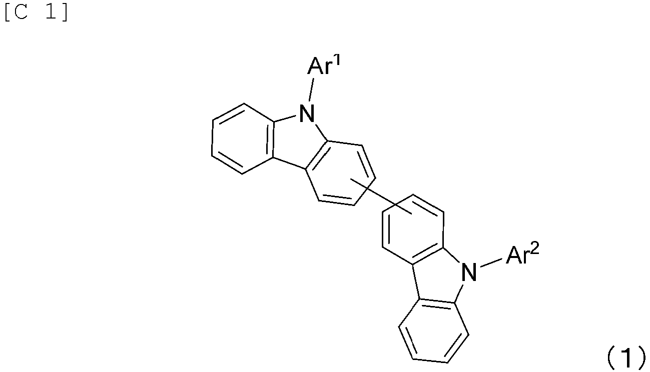 wherein Ar¹ and Ar² each independently represent a substituted or unsubstituted aromatic hydrocarbon group having 6 to 30 carbon atoms, or a substituted or unsubstituted linked aromatic group in which two to five of these aromatic rings are linked to each other, and aromatic hydrocarbon groups in the case of these aromatic rings linked are the same as or different from each other.

2. The deuteride according to claim 1, wherein the compound represented by the general formula (1) is a compound represented by the following formula (2): 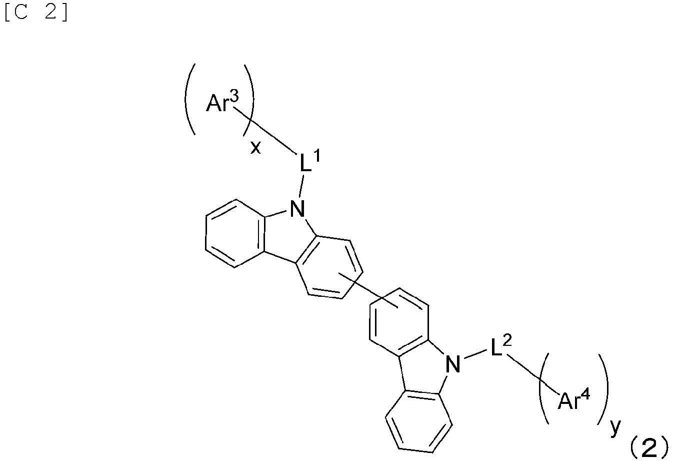 wherein Ar³ and Ar⁴ each independently represent a substituted or unsubstituted aromatic hydrocarbon group having 6 to 14 carbon atoms, or a substituted or unsubstituted linked aromatic group in which two of these aromatic rings are linked to each other, and aromatic hydrocarbon groups in the case of these aromatic rings linked are the same as or different from each other; L¹ and L² represent a substituted or unsubstituted phenylene group; and x and y each independently represent an integer of 0 to 2.

3. A mixture of the deuteride according to claim 1 and a compound represented by the following general formula (3) : 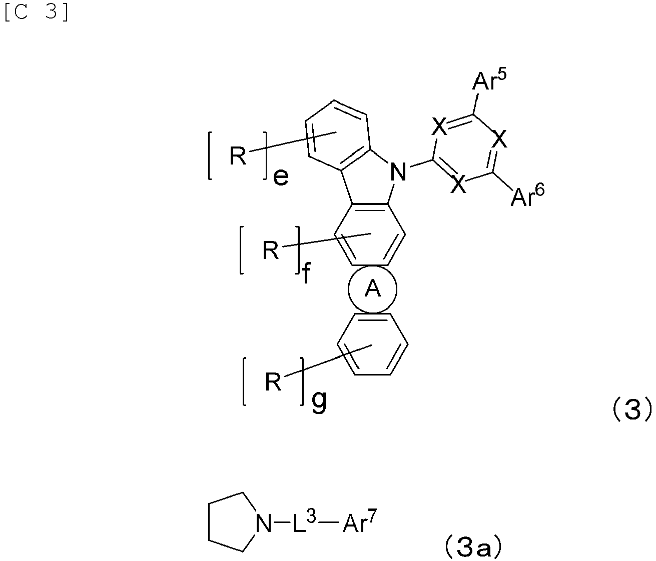 wherein a ring A is a heterocycle fused to two adjacent rings at any positions and represented by formula (3a); Ar⁵ and Ar⁶ each independently represent hydrogen, a substituted or unsubstituted aromatic hydrocarbon group having 6 to 30 carbon atoms, a substituted or unsubstituted aromatic heterocyclic group having 3 to 17 carbon atoms, or a substituted or unsubstituted linked aromatic group in which two to five of these aromatic rings are linked to each other, and aromatic hydrocarbon groups or aromatic heterocyclic groups in the case of these aromatic rings linked are the same as or different from each other; Ar⁷ represents a substituted or unsubstituted aromatic hydrocarbon group having 6 to 30 carbon atoms, a substituted or unsubstituted aromatic heterocyclic group having 3 to 17 carbon atoms, or a substituted or unsubstituted linked aromatic group in which two to five of these aromatic rings are linked to each other, and aromatic hydrocarbon groups or aromatic heterocyclic groups in the case of these aromatic rings linked are the same as or different from each other; L³ represents a direct bond or a substituted or unsubstituted aromatic hydrocarbon group having 6 to 18 carbon atoms; each R independently represents an aliphatic hydrocarbon group having 1 to 10 carbon atoms; e to g represent the number of substitutions, e and g represent an integer of 0 to 4, and f represents an integer of 0 to 2; X represents N, C-H, or C-Ar⁸ and at least one thereof represents N; and each Ar⁸ independently represents a substituted or unsubstituted aromatic hydrocarbon group having 6 to 30 carbon atoms, a substituted or unsubstituted aromatic heterocyclic group having 3 to 17 carbon atoms, or a substituted or unsubstituted linked aromatic group in which two to five of these aromatic rings are linked to each other, and aromatic hydrocarbon groups or aromatic heterocyclic groups in the case of these aromatic rings linked are the same as or different from each other.

4. The mixture according to claim 3, wherein a difference in 50% weight reduction temperatures of the deuteride according to claim 1 and the compound represented by the general formula (3) is within 20°C.

5. The mixture according to claim 4, wherein a proportion of the compound represented by the general formula (3) is 20 wt% or more and 70 wt% or less.

6. An organic electroluminescent device comprising a plurality of organic layers between an anode and a cathode, wherein at least one of the organic layers contains the deuteride according to claim 1 or the mixture according to claim 3.

7. The organic electroluminescent device according to claim 6, wherein the organic layer containing the deuteride or the mixture is at least one layer selected from the group consisting of a light-emitting layer, a hole injection layer, a hole transport layer, an electron transport layer, an electron injection layer, a hole blocking layer and an electron blocking layer.

8. The organic electroluminescent device according to claim 7, wherein the organic layer containing the deuteride or the mixture is a light-emitting layer, and the light-emitting layer contains at least one light-emitting dopant.

9. A method for producing an organic electroluminescent device comprising a plurality of organic layers between an anode and a cathode, wherein one of the organic layers is a light-emitting layer, and the light-emitting layer is produced by providing the mixture according to claim 3 and using the mixture for vapor-deposition from one vapor deposition source.
